# EUROPEAN PATENT SPECIFICATION

(11) **EP 4 017 976 B1**
(45) Date of publication and mention of the grant of the patent: **02.07.2025**
(21) Application number: 20756868.4
(22) Date of filing: 19.08.2020
(51) Int. Cl.: C12N 15/10, C12N 15/62, C12N 9/22, C12N 9/78, C12N 15/85, C12N 5/10, A61K 31/70, A61K 48/00

(54) **COILED-COIL MEDIATED TETHERING OF CRISPR/CAS AND EXONUCLEASES FOR ENHANCED GENOME EDITING**
DURCH SPIRALISIERTE SPIRALE VERMITTELTE BINDUNG VON CRISPR-CAS UND EXONUKLEASEN FÜR VERBESSERTE GENOMEDITIERUNG
CONNEXION À MÉDIATION DE SUPERHÉLICE DE CRISPR-CAS ET D'EXONUCLÉASES POUR AMÉLIORER L'ÉDITION DE GÉNOMES

(30) Priority: 20.08.2019 EP 19192490
(43) Date of publication of application: 29.06.2022
(73) Proprietor: Kemijski Institut, 1000 Ljubljana (SI)
(72) Inventor: JERALA, Roman, 1000 Ljubljana (SI); LAINSCEK, Dusko, 1000 Ljubljana (SI)
(74) Representative: Weickmann & Weickmann PartmbB
(86) International application number: PCT/EP2020/073143
(87) International publication number: WO 2021/032759

(56) References cited:
- WO-A1-2013/009525
- WO-A1-2014/089290
- WO-A1-2014/144288
- WO-A1-2015/089427
- WO-A1-2017/011721
- WO-A1-2017/053879
- WO-A1-2018/039438
- WO-A1-2018/176009
- WO-A1-2019/056002
- WO-A1-2019/123014
- WO-A1-2019/241649
- WO-A2-2010/132092
- WO-A2-2012/118717
- WO-A2-2015/035162
- WO-A2-2017/070633
- WO-A2-2019/139645
- Y BILL KIM ET AL: "Increasing the genome-targeting scope and precision of base editing with engineered Cas9-cytidine deaminase fusions", NATURE BIOTECHNOLOGY, vol. 35, no. 4, 13 February 2017 (2017-02-13), New York, pages 371 - 376, XP055484491, ISSN: 1087-0156, DOI: 10.1038/nbt.3803
- HOBO T ET AL: "A bZIP factor, TRAB1, ineracts with VP1 and mediates abscisic acid-induced transcription", PROCEEDINGS OF THE NATIONAL ACADEMY OF SCIENCES, NATIONAL ACADEMY OF SCIENCES, vol. 96, no. 26, 21 December 1999 (1999-12-21), pages 15348 - 15353, XP002964171, ISSN: 0027-8424, DOI: 10.1073/PNAS.96.26.15348
- NICOLE M GAUDELLI ET AL: "Programmable base editing of A.T to G.C in genomic DNA without DNA cleavage (Includes Methods)", vol. 551, no. 7681, 23 November 2017 (2017-11-23), pages 464 - 471, 16PP, XP002785203, Retrieved from the Internet <URL:http://www.nature.com/articles/nature24644> DOI: 10.1038/NATURE24644
- JOHN P GUILINGER ET AL: "Fusion of catalytically inactive Cas9 to FokI nuclease improves the specificity of genome modification", NATURE BIOTECHNOLOGY, vol. 32, no. 6, 1 June 2014 (2014-06-01), New York, pages 577 - 582, XP055157221, ISSN: 1087-0156, DOI: 10.1038/nbt.2909
- KUSCU CEM ET AL: "CRISPR-Cas9-AID base editor is a powerful gain-of-function screening tool", NATURE METHODS, NATURE PUBLISHING GROUP, GB, vol. 13, no. 12, 29 November 2016 (2016-11-29), pages 983 - 984, XP002793705, ISSN: 1548-7105, DOI: 10.1038/NMETH.4076
- KEENAN T ET AL: "Synthesis and activity of bivalent FKBP12 ligands for the regulated dimerization of proteins", BIOORGANIC & MEDICINAL CHEMISTRY, ELSEVIER, AMSTERDAM, NL, vol. 6, no. 8, 1 August 1998 (1998-08-01), pages 1309 - 1335, XP027388316, ISSN: 0968-0896, [retrieved on 19980801]
- YUAN JUANJUAN ET AL: "Genetic Modulation of RNA Splicing with a CRISPR-Guided Cytidine Deaminase", MOLECULAR CELL, ELSEVIER, AMSTERDAM, NL, vol. 72, no. 2, 4 October 2018 (2018-10-04), pages 380, XP085531578, ISSN: 1097-2765, DOI: 10.1016/J.MOLCEL.2018.09.002
- STEPHANIE VOß ET AL: "Chemically induced dimerization: reversible and spatiotemporal control of protein function in cells", CURRENT OPINION IN CHEMICAL BIOLOGY, vol. 28, 29 September 2015 (2015-09-29), GB, pages 194 - 201, XP055451688, ISSN: 1367-5931, DOI: 10.1016/j.cbpa.2015.09.003
- STÉPHANIE CABANTOUS ET AL: "A New Protein-Protein Interaction Sensor Based on Tripartite Split-GFP Association", SCIENTIFIC REPORTS, vol. 3, 4 October 2013 (2013-10-04), XP055260864, DOI: 10.1038/srep02854
- ALEXIS C. KOMOR ET AL: "Programmable editing of a target base in genomic DNA without double-stranded DNA cleavage", NATURE, vol. 533, no. 7603, 20 April 2016 (2016-04-20), pages 420 - 424, XP055551781, DOI: 10.1038/nature17946
- TAKAFUMI MIYAMOTO ET AL: "Rapid and orthogonal logic gating with a gibberellin-induced dimerization system", NATURE CHEMICAL BIOLOGY, vol. 8, no. 5, 1 January 2012 (2012-01-01), New York, pages 465 - 470, XP055221911, ISSN: 1552-4450, DOI: 10.1038/nchembio.922
- ROBERT DEROSE ET AL: "Manipulating signaling at will: chemically-inducible dimerization (CID) techniques resolve problems in cell biology", PFLUGERS ARCHIV - EUROPEAN JOURNAL OF PHYSIOLOGY, vol. 465, no. 3, 9 January 2013 (2013-01-09), Berlin/Heidelberg, pages 409 - 417, XP055221913, ISSN: 0031-6768, DOI: 10.1007/s00424-012-1208-6
- HUANG LIANGGANG ET AL: "Highly efficient single base editing inAspergillus nigerwith CRISPR/Cas9 cytidine deaminase fusion", MICROBIOLOGICAL RESEARCH, vol. 223, August 2019 (2019-08-01), pages 44 - 50, XP085706908, ISSN: 0944-5013, DOI: 10.1016/J.MICRES.2019.03.007
- BERND ZETSCHE ET AL: "A split-Cas9 architecture for inducible genome editing and transcription modulation", NATURE BIOTECHNOLOGY, vol. 33, no. 2, 2 February 2015 (2015-02-02), New York, pages 139 - 142, XP055227889, ISSN: 1087-0156, DOI: 10.1038/nbt.3149
- BERND ZETCHE ET AL.: "Supplementary Information: A Split Cas9 Architecture for Inducible Genome Editing and Transcription Modulation", 2 February 2015 (2015-02-02), pages 1 - 9, XP055661315, Retrieved from the Internet <URL:https://www.ncbi.nlm.nih.gov/pmc/articles/PMC4503468/bin/NIHMS702628-supplement-Materials.doc> [retrieved on 20200123]
- FINK TINA ET AL: "Design of fast proteolysis-based signaling and logic circuits in mammalian cells.", NATURE CHEMICAL BIOLOGY 02 2019 (ONLINE), vol. 15, no. 2, 10 December 2018 (2018-12-10), pages 115 - 122, XP002797138, ISSN: 1552-4469

## Description

### FIELD OF THE INVENTION

Heterodimerization of Cas9 protein, part of CRISPR/Cas system, with exonucleases via peptides that form coiled-coils increases the rate of occurrence of indel mutations in genome region of interests in cells resulting in higher degree of genome engineering that is exploited in all areas where genome editing is required.

### BACKGROUND OF THE INVENTION

The CRISPR (Clustered Regularly Interspaced Short Palindromic Repeats)/Cas (CRISPR-associated) system that is used as a tool for genome editing and derives from bacteria and archaea as a part of their adaptive immune system to fight infectious viruses. When prokaryotes are invaded with viruses, short viral DNA segments are integrated into CRISPR locus, which is the key for CRISPR immunity. Upon second accounter with viruses, RNA that includes viral sequence, from CRISPR locus is transcribed. That RNA is complimentary to viral DNA and mediates targeting of Cas9 protein to the sequence in genome. This specific targeting results in DNA cleavage and thereby silencing the viral target,
This specific targeting and DNA cleavage at desired genome region made CRISPR/Cas system as a potent novel tool for genome editing. Cas9 protein or its variants are guided by gRNA (guide RNA) to its complimentary specific site or region of interest (ROI) within the genome. The only necessity for Cas9 recognition is short PAM (protospacer adjacent motif) sequence presence within the genome. Depending on the Cas9 origin, different PAM sequences are recognized. After specific Cas9 targeting to ROI site-specific double strand breaks (DSBs) occur, which vary in length and in frequency. Repair of DSBs and their manipulation lead to knockout or knockin of gene of interests (GOI).

DSBs are then repaired with cell repair mechanisms, mostly with error-prone classical-Non-homologous end joining (c-NHEJ), which is DNA resection independent. Other repair mechanisms, like alt-EJ (alternative end joining), HR (homologous recombination), SSA (single-strand annealing) work in DNA resection dependent manner. C-NHEJ mainly introduces few nucleotides long insertion-deletion (indel) mutations that can lead to functional gene inactivation by either frameshift or deletions resulting in certain percentage or degree of knockout of GOI. C-NHEJ repairs Cas9 mediated blunt-end DSB via simple blunt end sequence-homology independent ligation process. Alternatively, DSBs can be resected, leaving 3' or 5' single-stranded DNA (ssDNA) overhangs. When DSBs via HR are repaired a certain DNA template as a source for repair is needed. Usually sister chromatid get strand invaded in typically error-free process, resulting in site-specific integration. This is often exploited for knockin production where included exogenous DNA reacts as a template. ssDNA overhangs can be also repaired via mutagenic repair mechanism, namely SSA, where annealing at large homologies occurs, resulting in larger deletions with no insertions. Alt-EJ uses annealing at microhomologies, resulting in mutagenic indel mutations.

Depending on the size and frequency of indel mutations, higher rates of knockout can be achieved. Greater genome rearrangements occurs when additional DNA resection after DSBs are carried out. DNA resection and subsequent DNA repair is used for elevated genome editing using CRISPR/Cas regarding the efficiency. Coexpression of certain exonucleases (e.g. human EXO1, TREX2) with CRISPR/Cas system can increase the size of mutations resulting in higher frequency of knockin or knockout of GOI.

### SUMMARY OF THE INVENTION

The present invention provides an implementation for CRISPR/Cas system for greater efficiency regarding genome modification and editing based on tethering of Cas9 protein with exonucleases via different heterodimerization systems. The invention is defined by the appended claims. Any other aspects described herein are provided for comparative purposes only. Tethering of CRISPR/Cas system and exonucleases result in higher percentage or degree of genome modification for GOI or recisse DNA to the extent to allow higher homologies for greater percentage of knockin.

This disclosure provides new system of improved genome editing by the use of CRISPR/Cas system. Genome editing herein encompasses DNA cleavage and sequential repair. DSBs are formed by protein Cas9. Also described but not claimed are Cas9 variants or Cas9 nickase (HNH-mutant nickase or RuvC-mutant nickase) that are guided via one or two gRNA to specific genome sites. This system for enhanced genome editing can be used in living mammalian and non-mammalian (e.g. plant) cells.

Heterodimerization of CRISPR/Cas system and certain exonucleases is achieved by the use of heterodimerizing peptides that form coiled-coils (CC) that are expressed with Cas9 protein at its C- or N- terminus and with certain exonucleases at its C- or N-terminus thus Cas9 gets tethered with certain exonucleases. According to the invention, the exonuclease is selected from Exol, Exolll and mTREX2. Tethering of Cas9 with certain exonucleases brings exonucleases into close proximity of DSB to influence DNA recession. Collocation of certain exonuclease at DSB improves DNA recession thus making longer 3' and/or 5' DNA overhangs. Longer DNA overhangs generates greater non-homologies between upper and lower DNA strands thus making larger indel mutations after DSB repairs at higher degree. Heterodimerization of Cas9 protein or its variants or Cas9 nickase and certain exonucleases is achieved due to the dimer forming peptides. According to the invention, hegerodimerizing proteins are orthogonal protein partners P3 and P4, or N5 and N6. Heterodimerizing peptides express coiled coil structural motif, a combination of hydrophobic and electrostatic interactions between amino acid residues within heptads of alpha-helices.

This disclosure presents tethering of CRISPR/Cas system and certain exonucleases via additional heterodimerization systems. System is based on the selection of heterodimerization (HD) domains (different protein-protein partners; HD system), which allows regulated expression in cells upon presence of one or more inductors that can provide external regulator signals. Inductors for heterodimerization can be light (e.g. Blue light, red light etc.) or chemical nature depending on the use of protein-protein partner pair. Such chemical inductors can be small molecules, such as rapalog, gibberellin and/or abscisic acid.

Protein partners of the HD system dimerize into a functional form when the appropriate heterodimerizing signal is present. One partner of the HD system is connected to a Cas9 protein. The other partner of the selected heterodimerization system is coupled with certain exonuclease. By the addition of an external light at the appropriate wavelength or small chemical inductor or regulator, respectively, (for example a rapalog, gibberellin or abscisic acid) that acts to promote heterodimerization, two partners of an HD system dimerize into a heterodimer. Heterodimerization of two protein partners of HD systems allows close proximity of certain exonuclease at the point of DSB, which is CRISPR/Cas system mediated. Juxtaposition of certain exonuclease at DSB enhances DNA recession thus making longer 3' and/or 5' DNA overhangs. Longer DNA overhangs generates greater non-homologies between upper and lower DNA strands thus making larger indel mutations after DSB repairs at higher degree.

The invention provides modified CRSIPR/Cas system based on the use of HD with certain exonucleases. Enhanced genome editing includes knockout and knockin for desired GOI at determined gene locus in mammalian and non-mammalian cells.

### Figure legends

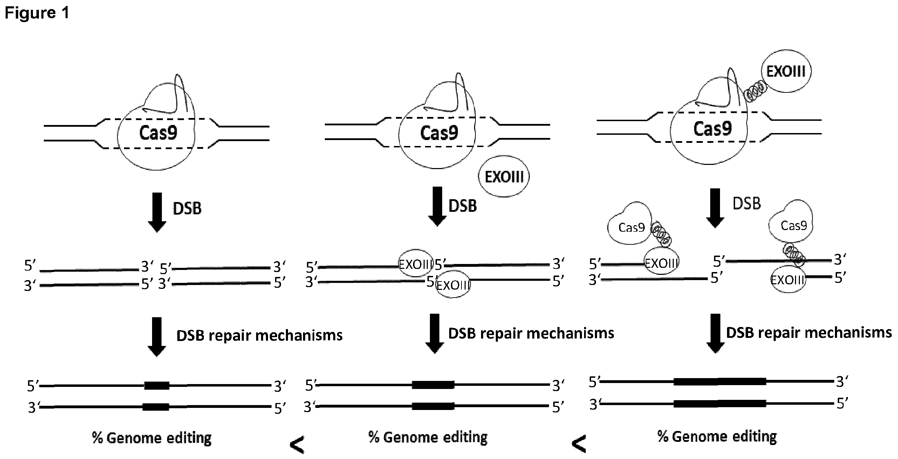

**Figure** 1: A schematic diagram of action of the method for enhanced genome engineering using CRISPR/Cas system according to the invention, tethered to exonucleases via heterodimeric peptides that form coiled-coils. A) By using simple CRISPR/Cas system that consists out of Cas9 protein and gRNA DSB are formed, which are then repaired via cell repair mechanisms, resulting in certain degree or percentage of genome editing. B) By using cooexpresion of simple CRISPR/Cas system and certain exonuclease (e.g. EXOIII) DSBs are formed. The exposed DNA strands are then additionally recised via certain exonucleases, creating DNA overhangs in 5' or 3' manner. Again, DSBs with established DNA overhangs are repaired via cell repair mechanisms, resulting in higher degree or percentage of genome editing compared to simple CRISPR/Cas system. C) By using CRISPR/Cas system tethered to certain exonuclease (e.g. EXOIII) via heterodimeric peptides that form coiled-coils DSBs are formed. The exposed DNA strands are then additionally recised via certain exonucleases, creating even larger DNA overhangs compared to overhangs, created in system, where CRISPR/Cas and exonucleases are coexpressed. Again, DSBs with established DNA overhangs are repaired via cell repair mechanisms, resulting in even higher degree or percentage of genome editing compared to simple CRISPR/Cas system or system, where CRISPR/Cas and exonucleases are coexpressed.

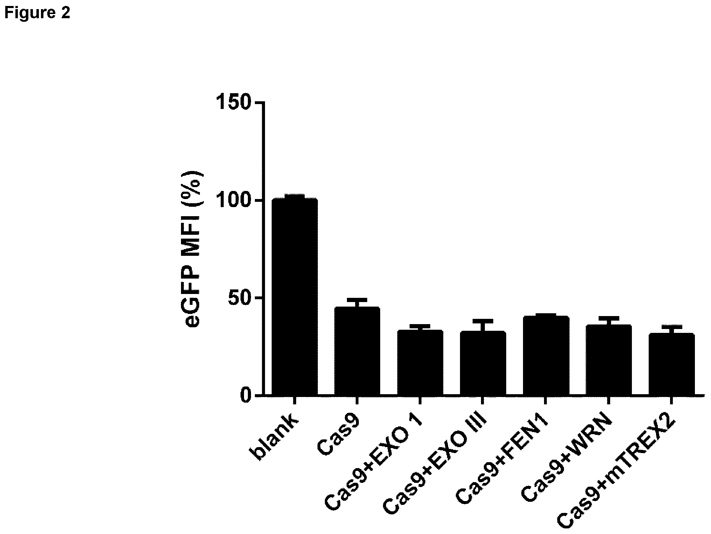

**Figure 2****:** Genome editing in eGFP genome region. By coexpression of Cas9 and gRNA, directed towards eGFP genomic region eGFP genome region is modified. Greater genome editing occurred when Cas9, gRNA were coeexpressed with exonucleases, wherein EXO1, EXOIII, FEN1, WRN or mTREX2 is at least one of the exonuclease used. eGFP genome editing resulted in eGFP fluorescence decrease. FEN1, and WRN are described as comparative examples only.

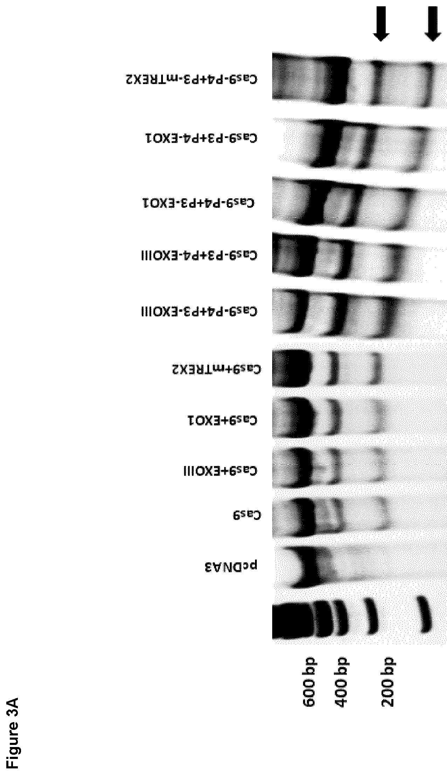

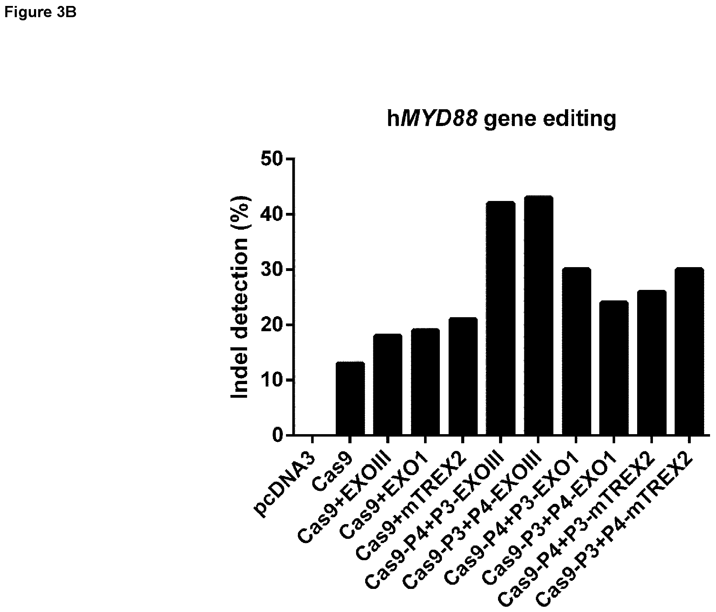

**Figure 3****:** Genome editing in human *MYD88* genome region. By coexpression of Cas9 and gRNA, directed towards human *MYD88* genomic region human *MYD88* genome region is modified. Greater genome editing occurred when Cas9, gRNA were coeexpressed with exonucleases, wherein EXO1, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EXO1, P4-EXO1, P3-EXOIII, P4-EXOIII, P3-mTREX2 or P4-mTREX2 are one of the variants of expressed exonucleases. A) Human *MYD88* genome editing resulted in indel mutations, detected via T7E1 assay. B) Indel detection (%) of *MYD88* genome region engineering.

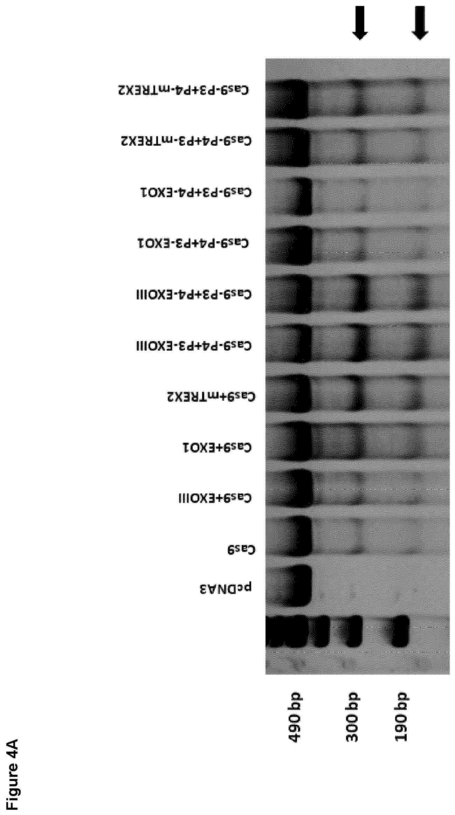

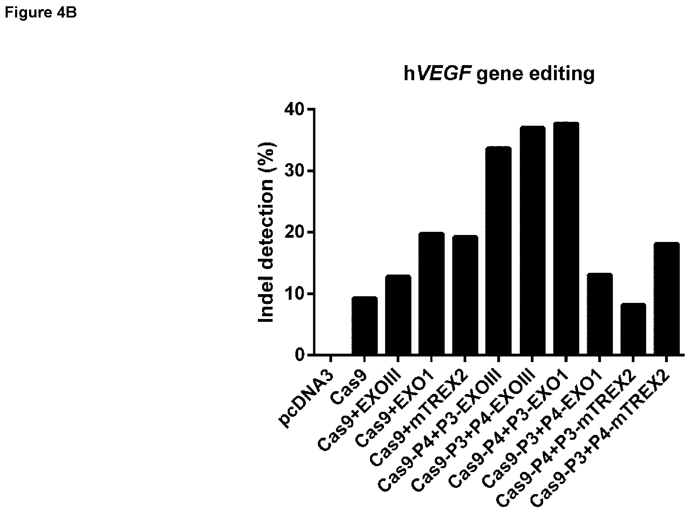

**Figure 4****:** Genome editing in human *VEGF* genome region. By coexpression of Cas9 and gRNA, directed towards human *VEGF* genomic region human *VEGF* genome region is modified. Greater genome editing occurred when Cas9, gRNA were coeexpressed with exonucleases, wherein EXO1, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EXO1, P4-EXO1, P3-EXOIII, P4-EXOIII, P3-mTREX2 or P4-mTREX2 are one of the variants of expressed exonucleases. A) Human *VEGF* genome editing resulted in indel mutations, detected via T7E1 assay. B) Indel detection (%) of *VEGF* genome region engineering.

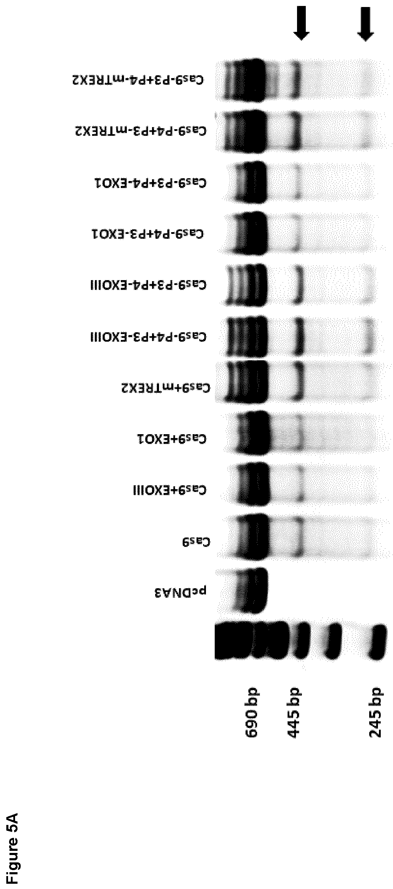

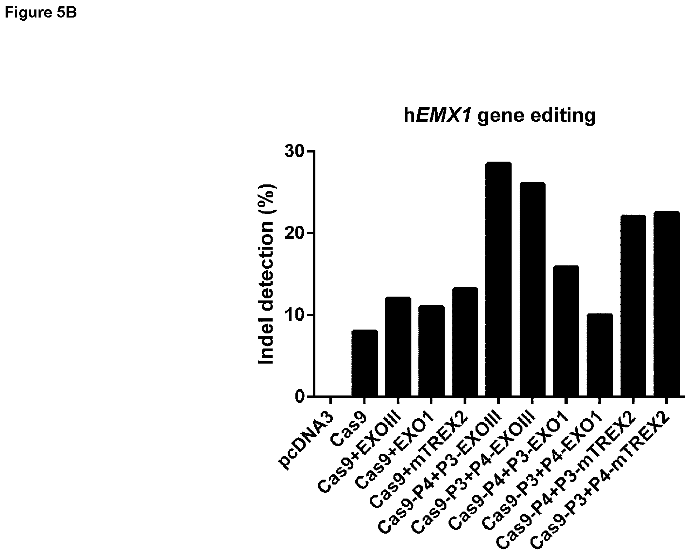

**Figure 5****:** Genome editing in human *EMX1* genome region. By coexpression of Cas9 and gRNA, directed towards human *EMX1* genomic region human *EMX1* genome region is modified. Greater genome editing occurred when Cas9, gRNA were coeexpressed with exonucleases, wherein EXO1, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EXO1, P4-EXO1, P3-EXOIII, P4-EXOIII, P3-mTREX2 or P4-mTREX2 are one of the variants of expressed exonucleases. A) Human *EMX1* genome editing resulted in indel mutations, detected via T7E1 assay. B) Indel detection (%) of *EMX1* genome region engineering.

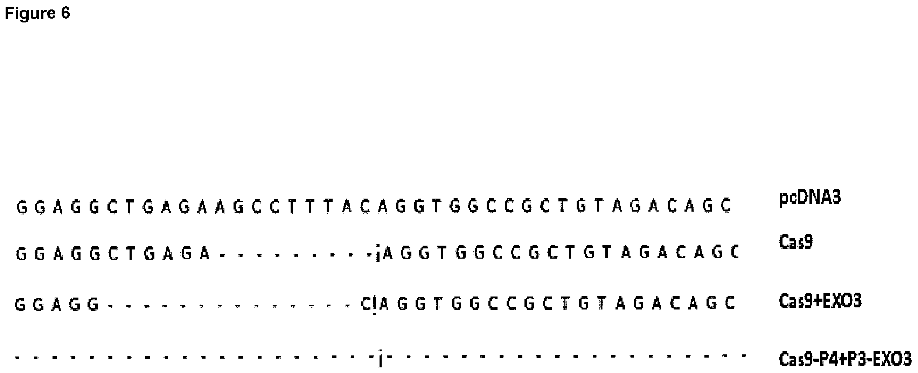

**Figure** 6: Equivalent Cas9 protein expression. Cas9 protein is expressed in the same manner when only Cas9 is expressed or cooexpressed with exonucleases, wherein EXO1, EXOIII, or mTREX2 is at least one of the exonuclease used. The same Cas9 expression is observed when CRISPR/Cas system tethered to certain exonucleases via heterodimeric coiled-coil forming peptides were used, wherein Cas9-P3 or Cas9-P4 are one of the Cas9 variants and P3-EXO1, P4-EXO1, P3-EXOIII, P4-EXOIII, P3-mTREX2 or P4-mTREX2 are one of the variants of expressed exonucleases. Cas9 expression is detected via Western blot and immunodetection.

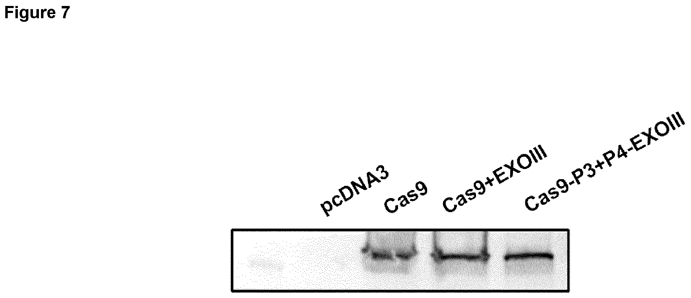

**Figure** 7: Genome editing in human *MYD88* genome region. By coexpression of Cas9 and gRNA, directed towards human *MYD88* genomic region human *MYD88* genome region is modified. Greater genome editing occurred when Cas9, gRNA were coeexpressed with exonucleases, wherein EXO1, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EXO1, P4-EXO1, P3-EXOIII, P4-EXOIII, P3-mTREX2 or P4-mTREX2 are one of the variants of expressed exonucleases. Next generation sequencing was performed to detect the size of edited genome region.

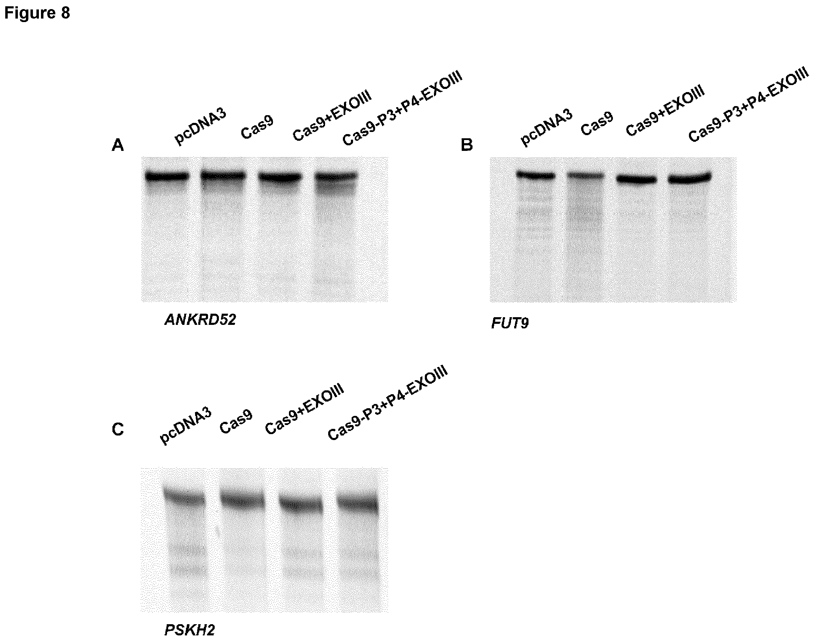

**Figure 8****:** Absence of human *MYD88* "off target" genome editing due to increased CRISPR/Cas action. By coexpression of Cas9 and gRNA, directed towards human *MYD88* genomic region human *MYD88* genome region is modified. Greater genome editing occurred when Cas9, gRNA were coeexpressed with exonucleases, wherein EXO1, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EXO1, P4-EXO1, P3-EXOIII, P4-EXOIII, P3-mTREX2 or P4-mTREX2 are one of the variants of expressed exonucleases. Three predicted coding regions of human *MYD88* gene targeting gRNA "off-target" sites were screened via T7E1 assay. A) Predicted "off-target" site 1 (human *ANKRD52* gene) genome editing did not result in indel mutations, detected via T7E1 assay. B) Predicted "off-target" site 2 (human *FUT9* gene) genome editing did not result in indel mutations, detected via T7E1 assay. C) Predicted "off-target" site 3 (human *PSKH2* gene) genome editing did not result in indel mutations, detected via T7E1 assay.

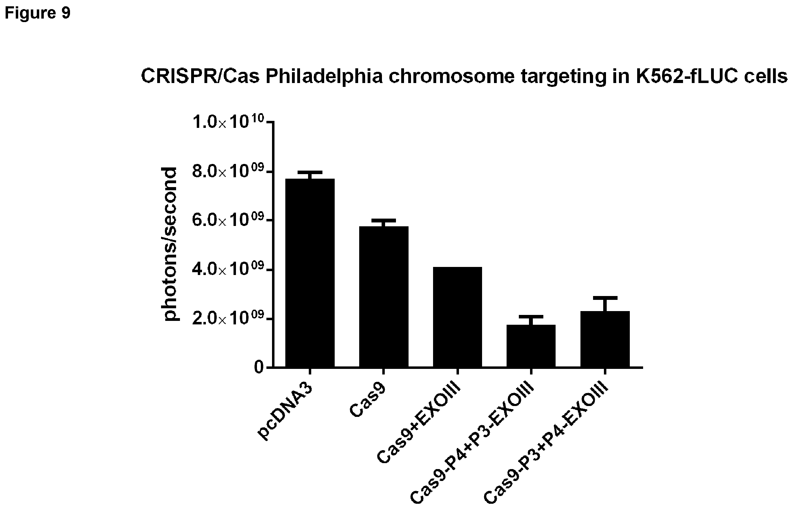

**Figure 9****:** Reduction in bioluminescence signal (photons/second) of K562-fLUC cells. gRNA targeting BCR-ABL chromosomal translocation was designed. CRISPR/Cas genome editing at BCR-ABL chromosomal translocation resulted in cell death, detected via drop of bioluminescence signal. By coexpression of Cas9 and gRNA, directed towards human BCR-ABL chromosomal translocation genomic region human *BCR-ABL* genome region is modified. Greater genome editing occurred when Cas9, gRNA were coeexpressed with exonucleases, wherein EXO1, EXOIII, or mTREX2 is at least one of the exonuclease used. Bigger genome editing occurred when CRISPR/Cas system tethered to certain exonucleases via heterodimeric coiled-coil forming peptides were used, wherein Cas9-P3 or Cas9-P4 are one of the Cas9 variants and P3-EXO1, P4-EXO1, P3-EXOIII, P4-EXOIII, P3-mTREX2 or P4-mTREX2 are one of the variants of expressed exonucleases.

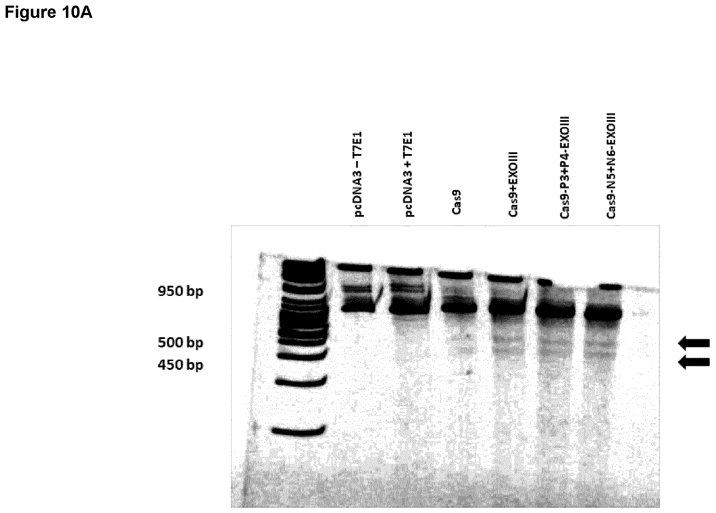

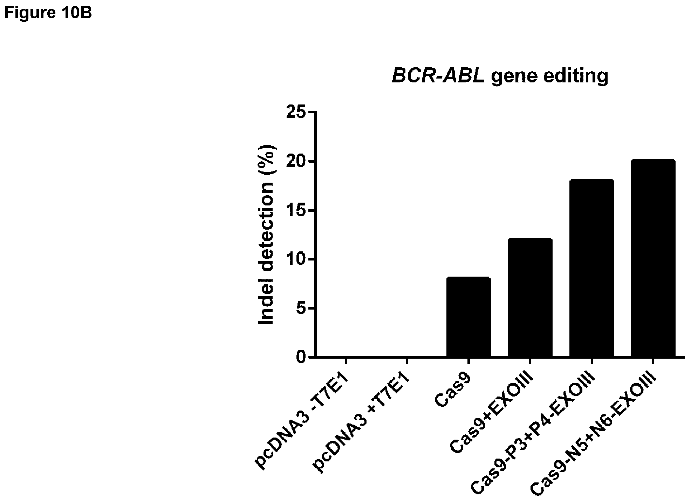

**Figure 10****:** Genome editing in human *BCR-ABL* genome region. By coexpression of Cas9 and gRNA, directed towards human *BCR-ABL* genomic region human *BCR-ABL8* genome region is modified. Greater genome editing occurred when Cas9, gRNA were coeexpressed with exonucleases, wherein EXOIII is at least one of the exonuclease used. Bigger genome editing occurred when CRISPR/Cas system tethered to certain exonucleases via heterodimeric coiled-coil forming peptides were used, wherein Cas9-P3 are one of the Cas9 variants and P4-EXOIII are one of the variants of expressed exonucleases. Even greater genome editing occurred when Cas9-N5 and N6-EXOIII variants were used, showing stronger heterodimerization affinity of peptides that form coiled-coils.

### Definitions

The term CRISPR/Cas system, as used herein relates to one or more gRNA that guides catalytically active Cas9 to the desired genomic site where DSB formations are formed through specific Cas9 action after appropriate PAM recognition. Cas9 protein in this invention is fused with heterodimerization system at N- or C-terminus that allows tethering with certain exonucleases.

Cas protein, as used herein relates to Cas9 and its variants and homologous derived from *Streptococcus pyogenes* that recognizes NGG or NAG PAM. For comparative purposes, this document additionally describes other DNA catalytically active or inactive endonucleases (dCas9) or nickases derived from all types of CRISPR system.

APOBEC polypeptide, as used herein relates to proteins that have deaminase activity and are connected with Cas9 and its variants and causes single base editing.

Genome editing, as used herein relates to any modification to genomic and/or nongenomic DNA due to CRISPR/Cas system and its efficiency to cause DSBs. Sequentially single base editing, knockin or knockout cells and/or organisms are processed.

Organism, as used herein relates to any living organism.

The term exonucleases as used herein, relates to any sort of enzymes that are able to cleave nucleotides from polynucleotide chain in 3' and/or 5' direction and are connected to heterodimerization system at N- or C-terminus.

The term "heterodimerization system", as used herein, refers to a pair of peptides or peptide domains that form coiled-coils or that connect in the presence of an inductor or a signal for dimerization. According to the invention, the heterodimerizing pair of peptides are orthogonal protein partners P3 and P4, or N5 and N6. The term "heterodimerization", as used herein, refers to protein domains or peptide pairs that connect to other domains of a different type through covalent or non-covalent interactions. The term "constitutive dimerization domains", as used herein, refers to dimerization domains that connect themselves independently, such as, for example, coiled coils. The term "inducible heterodimerization domain", as used herein, refers to heterodimerization domains that merge only in the presence of a heterodimerization inductor.

The term "heterodimerization inductor" refers to a signal, a chemical or non-chemical ligand, which cause the heterodimerization of protein domains that do not have intrinsic affinity with each other and cannot be connected independently in the absence of a heterodimedimerization ligand or signal. A heterodimerization inductor according to the invention can be a small molecule, such as rapalog, abscisic acid, or gibberellin. According to this invention, a heterodimerization inductor can also be light with certain wavelength.

Light induced heterodimerization system, as used herein, refers to protein partners CIB1 or its homologous and CRY2PHR, derived from *Arabidopsis thaliana* or are produced synthetically and heterodimerize in the presence of blue light with certain wavelength, approximately 450 nm. Another blue light induced heterodimerization system is LOVpep and ePDZB protein partners, derived from *Avena sativa.* This invention also relates to 650 nm wavelength red light induced heterodimerization system, where protein partners PIF and PHYB or its synthetic homologous are used. Another light induced heterodimerization system that this invention relies is heterodimerization of FKF1 and GI protein partners from *Gigantea* sp.

The term "chemical ligand", as used herein, refers to a small molecule, such as rapamycin and its rapalogs that can bind to proteins, preferably to FKBP-FRB heterodimerization domains; to plant hormones, such as abscisic acid and giberellin that can bind to proteins, preferably to ABI-PYL1 and GID-GAI1 heterodimerization domains.

The term peptide pair that form coiled coils, used herein refers to any peptide pair, heterodimerizing or homodimerizing peptides that express coiled coil structural motif, a combination of hydrophobic and electrostatic interactions between amino acid residues within heptads of alpha-helices that results in heterodimerization or homodimerization. According to the invention, the heterodimerizing pair of peptides are orthogonal protein partners P3 and P4, or N5 and N6.

The term "orthogonal", as used herein, refers to the characteristic of the components of the engineered CRISPR/Cas system tethered to certain exonucleases via different heterodimerization systems to take place separately, i.e., that the individual parts of different heterodimerization systems do not interact with parts of other heterodimerization systems of endogenous or exogenous signaling pathways. The main feature of the orthogonal system is that the input signal of each system leads to the heterodimerization independently of the presence or absence of other systems or their input and output signals.

The term "cell", as used herein, refers to an eukaryotic or prokaryotic cell, a cellular or multicellular organism (cell line) cultured as a single cell entity that has been used as a recipient of nucleic acids and includes the daughter cells of the original cell that has been genetically modified by the inclusion of nucleic acids. The term refers primarily to cells of higher developed eukaryotic organisms, preferably vertebrates, preferably mammals. This invention relies also on non-vertebrates cells, preferably plant cells.

The term "cells" also refers to human cell lines and plant cells. Naturally, the descendants of one cell are not necessarily completely identical to the parents in morphological form and its DNA complement, due to the consequences of natural, random or planned mutations. A "genetically modified host cell" (also "recombinant host cell") is a host cell into which the nucleic acid has been introduced. The eukaryotic genetically modified host cell is formed in such a way that a suitable nucleic acid or recombinant nucleic acid is introduced into the appropriate eukaryotic host cell. The invention hereafter includes host cells and organisms that contain a nucleic acid according to the invention (transient or stable) bearing the operon record according to the invention. Suitable host cells are known in the field and include eukaryotic cells. It is known that proteins can be expressed in cells of the following organisms: human, rodent, cattle, pork, poultry, rabbits and the like. Host cells may include cultured cell lines of primary or immortalized cell lines.

The term "nucleic acids", as used herein, refers to a polymeric form of nucleotides (ribonucleotides or deoxyribonucleotides) of any length and is not limited to single, double or higher chains of DNA or RNA, genomic DNA, cDNA, DNA-RNA hybrids, or polymers with a phosphorothioate polymer backbone made from purine and pyrimidine bases or other natural, chemical or biochemically modified, synthetic or derived nucleotide bases.

The term "protein", as used herein, refers to the polymeric form of amino acids of any length, which expresses any function, for instance localizing to a specific location, localizing to specific DNA sequence, facilitating and triggering chemical reactions, transcription regulation.

The term "recombinant", as used herein, means that a particular nucleic acid (DNA or RNA) is a product of various combinations of cloning, restriction and / or ligation leading to a construct having structurally coding or non-coding sequences different from endogenous nucleic acids in a natural host system.

The insertion of the vectors into the host cells is carried out by conventional methods known from the field of science, and the methods relate to transformation or transfection and include: chemically induced insertion, electroporation, micro-injection, DNA lipofection, cellular sonication, gene bombardment, viral DNA input, as well as other methods. The entry of DNA may be of transient or stable. Transient refers to the insertion of a DNA with a vector that does not incorporate the DNA of the invention into the cell genome. A stable insertion is achieved by incorporating DNA of the invention into the host genome. The insertion of the DNA of the invention, in particular for the preparation of a host organism having stably incorporated a nucleic acid, e.g. a DNA, of the invention, can be screened by the presence of markers. The DNA sequence for markers refers to resistance to antibiotics or chemicals and may be included on a DNA vector of the invention or on a separate vector.

The insertion of the CRISPR/Cas tethered with certain exonucleases via different heterodimerization systems are delivered to the cell as plasmid DNA or as mRNA or a proteins or as RNA:protein complexes where different parts of CRISPR/Cas system tethered with certain exonucleases via different heterodimerization systems can be as DNA or RNA or protein.

### DETAILED DESCRIPTION OF THE INVENTION

The invention relates to an enhanced CRISPR/Cas system as defined in the appended claims, that is made of a combination of two orthogonal protein partners of different heterodimerization (HD) systems or coiled-coil forming peptide pairs, connected with a Cas protein and certain exonucleases that reconstitute upon addition of an inductor of heterodimerization or via intrinsic affinity of peptide pairs that form coiled-coils. Functionally, enhanced CRISPR/Cas system influences the higher rate of genome editing based on larger insertion and/or deletion mutations that arise in higher percentage of genetically modified cells or organisms.

Chemical and non-chemical control over the activation of enhanced CRISPR/Cas system tethered to certain exonucleases via different heterodimerization systems of gene modified cells or organisms of this invention presents a novel way of gene editing in different research area, e.g. cancer therapy, new model organism production, new drug development etc.

The invention provides a combination comprising a first component (a) and a second component (b), wherein
a) the first component comprises a Cas9 endonuclease capable of inducing a double stranded DNA break, fused with a first protein or protein domain, and
b) the second component comprises an exonuclease selected from Exol, Exolll, and mTREX2, fused to a second protein or protein domain,
wherein the first and the second components are capable of heterodimerizing with each other via interaction of the first protein or protein domain and the second protein or protein domain.

The combination of the invention represents a modified CRISPR/Cas system. The endonuclease of the first component (a) can in particular be a Cas protein, a Cas nickase or dCas9 protein. The second component (b) can in particular be an exonuclease or cytidine deaminase, in particular APOBEC protein,
wherein the first protein or protein domain and the second protein or protein domain are orthogonal protein partners P3 and P4 or N5 and N6, forming a coiled-coil structure, in particular a coiled-coil structure in parallel or antiparallel orientation.

According to a particular aspect of the invention, heterodimerization of the first protein or protein domain and the second protein or domain is inducible via a chemical or non-chemical signal.

Addition of a chemical inductor of heterodimerization (HD) can trigger heterodimerization of the first component and the second component by heterodimerization of two protein partners (i.e. the at least one protein or protein domain and the second protein or protein domain), thereby resulting in a functional modified CRISPR/Cas system tethered with certain exonucleases, which can influence the genome modification of any GOI.

A chemical inductor for triggering heterodimerization of the at least one first protein or protein domain of the a) first component and the second protein or protein domain of the b) second component can be, e.g. rapalog (e.g. rapamycin) for triggering heterodimerization of a FKBP-FRB HD system (the at least one first protein domain is FKBP, the second protein domain is FRB), abscisic acid for triggering heterodimerization of a ABI-PYL1 HD system (the at least one first protein domain is ABI, the second protein domain is PYL1), gibberellin for triggering heterodimerization of a GID-GA1 HD system (the at least one first protein domain is GID, the second protein domain is GA1) or any other relevant physiological chemical signal.

Addition of a non-chemical inductor of heterodimerization (HD) can trigger heterodimerization of the first component and the second component by heterodimerization of two protein partners (i.e. the at least one protein or protein domain and the second protein or protein domain), thereby resulting in a functional modified CRISPR/Cas system tethered with certain exonucleases, which can influence the genome modification of any GOI.

A non-chemical inductor for triggering heterodimerization of the at least one first protein or protein domain of the a) first component and the second protein or protein domain of the b) second component can be light with appropriate wave length, e.g. blue light (e.g. 450 nm wave length) for triggering heterodimerization of a CIBN-CRY2PHR HD system (the at least one first protein domain is CIBN, the second protein domain is CRY2PHR) or LOVpep-ePDZb HD system (the at least one first protein domain is LOVpep, the second protein domain is ePDZb). Other inductor for light induced heterodimerization is red light (e.g. 650 nm wave length) for triggering heterodimerization of a PIF-PHYB HD system (the at least one first protein domain is PIF, the second protein domain is PHYB) or for triggering heterodimerization of a FKF1-GI HD system (the at least one first protein domain is FKF1, the second protein domain is GI) or any other relevant physiological non-chemical signal.

In a further embodiment of the invention, the heterodimerization between endonuclease and certain exonuclease can be achieved using peptide pairs that form coiled-coils due to the intrinsic affinity and electrostatic interactions and thus bringing certain exonucleases into close proximity of DSB allowing exonuclease to additionally recise DNA in 3' and/or 5' manner. In this embodiment, enhancing the function of regular CRISPR/Cas system through additional DNA recizing with certain exonucleases can result in greater percentage of genome modification compared to normal CRISPR/cas system or system where CRISPR/Cas and certain exonucleases are cooexpressed in tested cell or organism (see, e.g. Fig. 1).

The first component of peptide pair coiled-coil induced heterodimerization system is at least one peptide or peptide domain from peptide pair that form coiled-coils of a selected peptide pairs (i.e. P3-P4, or N5-N6) that is genetically fused to a Cas protein; for example: P3, N5 or P3S can be connected to Cas9 protein .

The second component of peptide pair coiled-coil induced heterodimerization system is at least one peptide or peptide domain from peptide pair that form coiled-coils of a selected peptide pairs (i.e. P3-P4, or N5-N6) that is genetically fused to a certain exonucleases (human EXO1, EXOIII E.coli, TREX2).

In a further embodiment of the invention, the selected pair of peptide pair that form coiled-coils are chosen based on their physical properties (e.g. Kd values, their orthogonality properties etc.). Peptide pairs that form coiled-coils can have strong heterodimerization properties: for example, peptide pair N5-N6 exhibit stronger heterodimerization affinity than peptide pair P3S-P4S. From this follows that percentage of genome editing in N5-N6 used heterodimerizing peptide pair that form coiled-coils for enhanced CRISPR/Cas system tethered with certain exonucleases is greater than for the example where P3S-P4S heterodimerizing peptide pair that form coiled-coils is used, that exhibit poor heterodimerization properties.

In a further embodiment, heterodimerization of peptide pairs, which form coiled-coils, can be obtained by using peptide pairs that heterodimerize in parallel (e.g. N5-N6, P3-P4 etc.) or anti-parallel (e.g.P3-AP4) manner.

In a further embodiment the use of heterodimerization systems for tethering CRISPR/Cas system with certain exonucleases, additional DNA recession with certain exonucleases and sequential improvement in genome editing does not enhance possible off-target activities of CRISPR/Cas system.

In a further embodiment, the modified CRISPR/Cas system tethered to certain exonucleases via different heterodimerization systems according to the invention comprises several repeats of the at least one first protein domain and/or the second protein domain, e.g. 10 repeats of a coiled coil. In this embodiment, the genome editing can be further enhanced. Several repeats (for example 10 repeats of coiled coil of a peptide pair) of the at least one first protein domain can then bind to several repeats (for example 10 repeats of coiled coil) of the second protein domain, which is fused to certain exonuclease. The first component comprising the at least one first protein domain having several repeats then heterodimerizes with the second component comprising the second protein domain having several repeats after the appropriate chemical inductor is added. In this embodiment, the repeats can act as constitutive dimerization domains that assemble upon the heterodimerization signal by the chemical inductor as orthogonal coiled coil pairs.

The invention further provides an isolated nucleic acid molecule, or a combination of isolated nucleic acid molecules, comprising a first nucleic acid sequence encoding the first component (a) and a second nucleic acid sequence encoding the second component (b) of the combination according to the invention as defined above. The invention further provides a vector, preferably an expression vector, encoding the nucleic acid molecule(s) of the invention.

In particular, the invention provides isolated nucleic acid molecules comprising sequences comprising or consisting of SEQ ID NOs: 1-18, as per the enclosed sequence listing. The invention further provides vectors comprising nucleic acid sequences comprising or consisting of SEQ ID NOs: 1-18, as per the enclosed sequence listing.

The invention further provides a vector encoding the isolated nucleic acids for protein isolation of the invention.
The invention further provides a cell or organism comprising the combination, the nucleic acid molecule(s) or the vector according to the invention as defined above, provided that the cell is not a human germ line cell.

The invention further provides the combination, the nucleic acid molecule(s) or the vector according to the invention as defined above for use in medicine, or for use as a medicament. The invention further provides the combination, the nucleic acid molecule(s) or the vector according to the invention as defined above for use in a method of treating cancer. In one embodiment of the invention, the aforementioned use comprises the use of a chemical inductor of heterodimerization. Preferably, the cells for use in a method of treating cancer are modified T cells. Preferably, the types of cancer to be treated according to the aforementioned use according to the invention include, CML, B-cell lymphoma, acute lymphoblastic leukemia, chronic lymphocytic leukemia, Hodgkins and Non-Hodgkins Lymphomas, mezotelioma, ovarian cancer, prostate cancer etc..

The present disclosure further describes an in vitro method for making knockout cells or organisms in more efficient manner where invention provides the combination, the nucleic acid molecule(s) or the vector according to the invention as defined above where genome editing is enhanced by the tethered exonucleases.

The present disclosure further describes an in vitro method for making knockin cells or organisms in more efficient manner where invention provides the combination, the nucleic acid molecule(s) or the vector according to the invention as defined above where genome editing is enhanced by the tethered exonucleases and by coaddition of template DNA.

The present disclosure further describes the combination, the nucleic acid molecule(s) or the vector according to the invention as defined above for use as a tool for drug new sources of fuel development etc.
The examples described in more detail below are designed to best describe the invention. These examples do not limit the scope of the invention, but are merely intended to provide a better understanding of the invention and its use.

### Example 1: Preparation of constructs for modified CRISPR/Cas system tethered with certain exonucleases via different heterodimerization systems

*Preparation of DNA coding for modified CRISPR*/*Cas system tethered with certain exonucleases via different heterodimerization systems.* In order to prepare DNA constructs, the inventors used molecular biology methods such as: chemical transformation of competent E. *coli* cells, plasmid DNA isolation, polymerase chain reaction (PCR), reverse transcription - PCR, PCR linking, nucleic acid concentration determination, DNA agarose gel electrophoresis, isolation of fragments of DNA from agarose gels, chemical synthesis of DNA, DNA restriction with restriction enzymes, cutting of plasmid vectors, ligation of DNA fragments, purification of plasmid DNA in large quantities. The exact course of experimental techniques and methods are well known to experts in the field and are described in the manuals of molecular biology.

All the work was performed using sterile techniques, which are also well known to the experts in the field. All plasmids, completed constructs and partial constructs were transformed into the bacteria *Escherichia coli* by chemical transformation. Plasmids for transfection into cell lines (animal or human) have been isolated using a DNA isolation kit that removes endotoxins.

In the described cases, the Cas9 protein was selected from all types of CRISPR system. Exonucleases were selected among known exonucleases. The Cas9 was connected via a peptide linker with different protein domains of different heterodimerization systems or with peptide from peptide pairs that form coiled-coils at C-terminus. Exonucleases were connected via a peptide linker with different protein domains of different heterodimerization systems or with peptide from peptide pairs that form coiled-coils at N-terminus.

The sequences of Cas9 and certain exonucleases according to the invention are listed in **Table 1.** All operons were prepared by techniques according to methods known to experts in the field. The operons were inserted into plasmids suitable for eukaryotic systems. The suitability of the nucleotide sequence was confirmed by the inventors by sequencing and restriction analysis.

**Table 1: Fusion proteins of components of exemplary artificial engineered NFAT2 transcription factors.**

| | | | |
|---|---|---|---|
| 1 | Cas9-P4 | pcDNA3 | Cas9 from *Streptococcus pyogenes* fused with P4 peptide, that form coiled-coil |
| 2 | Cas9-P3 | pcDNA3 | Cas9 from *Streptococcus pyogenes* fused with P3 peptide, that form coiled-coil |
| 3 | Cas9-N5 | pcDNA3 | Cas9 from *Streptococcus pyogenes* fused with N5 peptide, that form coiled-coil |
| 4 | Cas9-N6 | pcDNA3 | Cas9 from *Streptococcus pyogenes* fused with N6 peptide, that form coiled-coil |
| 5 | Cas9-P3S | pcDNA3 | Cas9 from *Streptococcus pyogenes* fused with P3S peptide, that form coiled-coil |
| 6 | Cas9-P4S | pcDNA3 | Cas9 from *Streptococcus pyogenes* fused with P4S peptide, that form coiled-coil |
| 7 | Cas9-AP4 | pcDNA3 | Cas9 from *Streptococcus pyogenes* fused with AP4 peptide, that form coiled-coil |
| 8 | P4-EXOIII | pcDNA3 | Exonuclease EXOIII, derived from *Escherichia coli* fused with P4 peptide, that form coiled-coil |
| 9 | P3-EXOIII | pcDNA3 | Exonuclease EXOIII, derived from *Escherichia coli* fused with P3 peptide, that form coiled-coil |
| 10 | N5-EXOIII | pcDNA3 | Exonuclease EXOIII, derived from *Escherichia coli* fused with N5 peptide, that form coiled-coil |
| 11 | N6-EXOIII | pcDNA3 | Exonuclease EXOIII, derived from *Escherichia coli* fused with N6 peptide, that form coiled-coil |
| 12 | P3S-EXOIII | pcDNA3 | Exonuclease EXOIII, derived from *Escherichia coli* fused with P3S peptide, that form coiled-coil |
| 13 | P4S-EXOIII | pcDNA3 | Exonuclease EXOIII, derived from *Escherichia coli* fused with P4S peptide, that form coiled-coil |
| 14 | AP4-EXOIII | pcDNA3 | Exonuclease EXOIII, derived from *Escherichia coli* fused with AP4 peptide, that form coiled-coil |
| 15 | P3-TREX2 | pcDNA3 | Exonuclease TREX2, derived from *Mus musculus* fused with P3 peptide, that form coiled-coil |
| 16 | P4-TREX2 | pcDNA3 | Exonuclease TREX2, derived from *Mus musculus* fused with P4 peptide, that form coiled-coil |
| 17 | P3-EXO1 | pcDNA3 | Exonuclease EXO1, derived from *Homo sapiens* fused with P3 peptide, that form coiled-coil |
| 18 | P4-EXO1 | pcDNA3 | Exonuclease EXO1, derived from *Homo sapiens* fused with P4 peptide, that form coiled-coil |

Methods and techniques of cultivating cell cultures are well known to experts in the field and are therefore briefly described in order to illustrate the implemented examples. Cells from the HEK293T cell line were grown at 37 ° C and 5% CO₂. For cultivation, a DMEM medium containing 10% FBS was used, containing all the necessary nutrients and growth factors. Cells K562 were grown in RPMI medium containing 10% FBS at 37 ° C and 5% CO₂. When the cell culture reached the appropriate density, the cells were grafted into a new breeding flask and / or diluted. For the use of cells in experiments, the number of cells was determined by a hemocytometer and seeded in density 1x10⁵ per hole into the microtiter plate with 24 holes 18-24 hours before transfection regarding HEK293 cells. The seeded plates were incubated at 37 ° C and 5% CO₂ until the cells were 50-70% confluent and ready for transfection by transfection reagent. The transfection was carried out according to the instructions of the transfection reagent manufacturer (e.g., JetPei, Lipofectamine 2000) and was adapted for the microtiter plate used. K562 (1x10⁴) were electroporated with the use of Neon electroporation system in 10 µl electroporation tips. Afterwards the cells were put in RPMI medium, containg 10% FBS in a well of 12 well plate. On the next day the inductor for heterodimerization were added if chemical and non-chemical induced heterodimerization system were used.48 hours after transfection genomic DNA was isolated and desired area PCR amplified. Next T7E1 (T7 endonuclese 1) assay was performed to determine the percentage of indel mutations.

### Example 2. Defining the most successful exonuclease cooexpressed with CRISPR/Cas system for enhacing genome editing at eGFP genomic region

To detect the most efficient exonuclease cooexpression of certain exonucleases with CRISPR/Cas system was used. HEK293 cell line that stably expresses eGFP was used.

HEK293T, stably expressing eGFP cells, seeded in 24-well plates, were transfected one day prior to the experiment with plasmids, which encode for one of the examined exonuclease and a separate plasmid that encodes CRISPR/Cas system; Cas9 protein from *Streptococcus pyogenes* and gRNA (targeting sequence in eGFP genomic region: *GGCGAGGGCGATGCCACCTA*).

To analyze the genome editing activity of cooexpression of certain exonuclease and CRISPR/Cas system, cells were analyzed on flow cytometry 48 hours after transfection. We measured the fluorescence intensity in cell population respectively and presented results in % of MFI (mean fluorescence intensity)

### Results:

It is clear from Figure 2 that the cooexpression of certain exonucleases and CRISPR/Cas system did enhance genome editing in eGFP genomic region regarding diminished fluorescence in all cell samples, transfected with CRISPR/Cas system and certain exonuclease. Cooexpression of CRISPR/Cas system targeting *eGFP* GOI and EXOIII, derived from *E.coli* showed the most edited percentage of tested cells due to the lowest % of MOI.

### Example 3. Activity of modified CRISPR/Cas system tethered with certain exonucleases via heterodimerizing peptide pairs that form coiled-coils

To test the activity of modified CRISPR/Cas system tethered with certain exonucleases via heterodimerizing peptide pairs HEK293 cells were used to determine the activity of modified CRISPR/Cas system tethered with certain exonucleases via heterodimerizing peptide pairs. Cells were transfected with constructs that express CRISPR/Cas system tethered with certain exonucleases via heterodimerizing peptide pairs; for example Cas9-P3 or Cas9-P4 with combination of P3-EXO1 or P4-EXO1 or P3-EXOIII or P4-EXOIII or P3-mTREX2. For control, cells were transfected with regular CRISPR/Cas9 system or were cooexpressed with CRISPR/Cas9 system and certain exonuclease (e.g. EXO1 or EXOIII or mTREX2). gRNA for human *EMX1* gene (targeting sequence: *GAGTCCGAGCAGAAGAAGAA*) or human *VEGF* gene (targeting sequence: *GGTGAGTGAGTGTGTGCGTG*) or human *MYD88 (GGCTGAGAAGCCTTTACAGG*) were used. 48 hours genomic DNA from transfected cells was isolated and genomic region around predicted DSB was PCR amplified. Next T7E1 assay was carried out. Presence and quantification of indel mutation was determined by Syber gold stained PAGE (polyacrylamide gel electrophoresis) gel analysis. Percentage of indel mutations was calculated by ImageJ software. Also next-generation sequencing (NGS) was performed from 150 base pair long amplicons.

### Result:

PAGE gel analysis of T7E1 treated samples from transfected cells with appropriate constructs in Figure 3A indicate that genome editing of human *MYD88* gene was highest among all tested samples when Cas9-P4 tethered to EXOIII via P3 peptide was used. A modest decrease in genome editing was observed when cells were transfected with constructs, expressing Cas9-P3 and P4-EXOIII. In all cases, where modified CRISPR/Cas system tethered with certain exonucleases (EXO1 or EXOIII or mTREX2) via heterodimerizing peptide pairs (P3-P4) were used genome editing was higher compared to cell samples with cooexpression of Cas9, gRNA targeting human *MYD88* gene and certain exonucleases (e.g. EXO1 or EXOIII or mTREX2). Genome editing, where modified CRISPR/Cas system tethered with certain exonucleases (EXO1 or EXOIII or mTREX2) via heterodimerizing peptide pairs (P3-P4) were used was also higher when regular CRISPR/Cas system was used, where only Cas9 and gRNA targeting human *MYD88* gene were used. Figure 3B graphically shows percentage of indel presence, which were determined with PAGE analysis, depicted in figure 3A.

PAGE gel analysis of T7E1 treated samples from transfected cells with appropriate constructs in Figure 4A indicate that genome editing of human *VEGF* gene was highest among all tested samples when Cas9-P3 tethered to EXOIII via P4 heterodimerizing peptide was used. A modest decrease in genome editing was observed when cells were transfected with constructs, expressing Cas9-P4 and P3-EXOIII. Genome editing was higher compared to cell samples with cooexpression of Cas9, gRNA targeting human *VEGF* gene and certain exonucleases (e.g. EXO1 or EXOIII or mTREX2). Genome editing, where modified CRISPR/Cas system tethered with certain exonucleases (EXO1 or EXOIII) via heterodimerizing peptide pairs (P3-P4) were used was also higher when regular CRISPR/Cas system was used, where only Cas9 and gRNA targeting human *VEGF* gene were used. Figure 4B graphically shows percentage of indel presence, which were determined with PAGE analysis, depicted in figure 4A.

PAGE gel analysis of T7E1 treated samples from transfected cells with appropriate constructs in Figure 5A indicate that genome editing of human *EMX1* gene was highest among all tested samples when Cas9-P4 tethered to EXOIII via P3 peptide was used. A modest decrese in genome editing was observed when cells were transfected with constructs, expressing Cas9-P3 and P4-EXOIII. In all cases, where modified CRISPR/Cas system tethered with certain exonucleases (EXO1 or EXOIII or mTREX2) via heterodimerizing peptide pairs (P3-P4) were used genome editing was higher compared to cell samples with cooexpression of Cas9, gRNA targeting human *EMX1* gene and certain exonucleases (e.g. EXO1 or EXOIII or mTREX2). Genome editing, where modified CRISPR/Cas system tethered with certain exonucleases (EXO1 or EXOIII or mTREX2) via heterodimerizing peptide pairs (P3-P4) were used was also higher when regular CRISPR/Cas system was used, where only Cas9 and gRNA targeting human *EMX1* gene were used. Figure 5B graphically shows percentage of indel presence, which were determined with PAGE analysis, depicted in figure 5A.

Figure 6 shows results from NGS. 150 base pair PCR amplicons from cells that were transfected with empty vector pcDNA3 or with regular Cas9 or with Cas9, cooexpressed with EXOIII or with Cas9-P4 and P3-EXOIII were sequenced. In all cases gRNA targeting human *MYD88* gene was used. No base pair deletion was observed in sample where only pcDNA3 was expressed. Nine base pair deletion was observed in sample where only Cas9 and gRNA was expressed. Fourteen base pair deletion was observed in sample where Cas9 and gRNA was coexpressed with EXOIII. Forty base pair deletion was observed in sample where Cas9-P4 tethered with P3-EXOIII and gRNA was expressed. Sign (i) depicts the predicted cleavage site for Cas9.

### Example 4: Genome editing of modified CRISPR/Cas system tethered with certain exonucleases via heterodimerizing peptide pairs is not due to the higher Cas9 protein expression

Higher percentage of genome editing for desired GOI can be enhanced due to higher cas9 protein expression. Genome editing of modified CRISPR/Cas system tethered with certain exonucleases via heterodimerizing peptide pairs is not due to the higher Cas9 protein expression.

To experimentally determine that higher genome editing of modified CRISPR/Cas system tethered with certain exonucleases via heterodimerizing peptide pairs is not due to Cas9 protein overexpression we visualized Cas9 protein content via western blot and anti-Cas9 specific immunodetection. To show the same Cas9 protein expression, HEK293 cells were transfected with constructs that express empty pcDNA3 plasmid, CRISPR/Cas system tethered with certain exonucleases via heterodimerizing peptide pairs; for example Cas9-P3 with combination of P4-EXOIII. For control, cells were transfected with regular CRISPR/Cas9 system or were cooexpressed with CRISPR/Cas9 system and certain exonuclese (e.g. EXOIII). gRNA for human *MYD88* (target sequence: *GGCTGAGAAGCCTTTACAGG*) was used. 48 hours cell lysates were prepared. The same amount of protein, determined by standard BCA assay, was analyzed on SDS PAGE gel analysis. Next, western blot transfer was performed and afterwards the membrane was stained with specific anti-Cas9 antibodies.

### Result:

Figure 7 shows that Cas9 protein content is equal in all tested samples. The band intensity of Cas9 protein is the same for cell lysates of cells, which were transfected with regular CRISPR/Cas9 system, where only Cas9 and gRNA are expressed or for cells that were used for cooexpression of Cas9 and certain exonuclease (e.g. EXOIII) and gRNA and for cells that were transfected with CRISPR/Cas system tethered with certain exonucleases via heterodimerizing peptide pairs; for example Cas9-P3 with combination of P4-EXOIII. In sample where only empty vector was transfected Cas9 protein band is absent.

### Example 5: Higher genome editing due to the modified CRISPR/Cas system tethered with certain exonucleases via heterodimerizing peptide pairs does not increase unwanted genome modification at off-target sites

When using CRISPR/Cas system with enhanced functionality there are some possibilities that increased on-target action of CRISPR/Cas bears also additional amplified off-target effect. To show that the modified CRISPR/Cas system tethered with certain exonucleases via heterodimerizing peptide pairs does not increase unwanted genome modification at off-target sites HEK293 cells where transfected with designated constructs for CRISPR/Cas. With standard T7E1 assay 48 hours after transfection potential three off-target sites for *MYD88* gRNA (target sequence: *GGCTGAGAAGCCTTTACAGG*) were analyzed. Due to bioinformatics tool for gRNA design and scoring three potential off-target sites for *MYD88* gRNA were predicted, for example: off-target site 1 was found in *ANKRD52* gene (target sequence: *ACTGTAAAGGCTGCTCTCCC*); off-target site 2 was predicted to be in *FUT9* gene (target sequence: *TGCAGAGGAGCCTTTACATG*) and off-target site 3 was determined in *PSKH2* gene (target sequence: *GCCAGACAAGGCTTTACAGG*).

### Result:

PAGE gel analysis of T7E1 treated samples from transfected cells with appropriate constructs depicted in Figure 8 indicate that genome editing of three predicted possible off-target sites for human *MYD88* gene gRNA (target sequence: *GGCTGAGAAGCCTTTACAGG*) did not happen. In figure 8A PAGE gel analysis of T7E1 treated samples from transfected cells for off-target site 1 that was found in *ANKRD52* gene (target sequence: *ACTGTAAAGGCTGCTCTCCC*) is depicted. There is no indel mutation present due to the absence of DNA bands cleavage. There is no DNA bands cleavage in samples, which were transfected with empty (pcDNA3) vector or with regular CRISPR/Cas9 system, where only Cas9 and gRNA are expressed. No DNA bands cleavage is seen also in cells, that were used for cooexpression of Cas9 and certain exonuclease (e.g. EXOIII) and gRNA and for cells that were transfected with CRISPR/Cas system tethered with certain exonucleases via heterodimerizing peptide pairs; for example Cas9-P3 with combination of P4-EXOIII.

In figure 8B PAGE gel analysis of T7E1 treated samples from transfected cells for off-target site 2 that was found in *FUT9* gene (target sequence: *TGCAGAGGAGCCTTTACATG*) is depicted. There is no indel mutation present due to the absence of DNA bands cleavage. There is no DNA bands cleavage in samples, which were transfected with empty (pcDNA3) vector or with regular CRISPR/Cas9 system, where only Cas9 and gRNA are expressed. No DNA bands cleavage is seen also in cells, that were used for cooexpression of Cas9 and certain exonuclease (e.g. EXOIII) and gRNA and for cells that were transfected with CRISPR/Cas system tethered with certain exonucleases via heterodimerizing peptide pairs; for example Cas9-P3 with combination of P4-EXOIII.

In figure 8C PAGE gel analysis of T7E1 treated samples from transfected cells for off-target site 3 that was found in *PSKH2* gene (target sequence: *GCCAGACAAGGCTTTACAGG*) is depicted. There are DNA bands cleavage present, but there is also DNA bands cleavage in samples, which were transfected with empty (pcDNA3) vector or with regular CRISPR/Cas9 system, where only Cas9 and gRNA are expressed, thus suggesting presence of SNP in the tested region. DNA bands cleavage are seen also in cells, that were used for cooexpression of Cas9 and certain exonuclease (e.g. EXOIII) and gRNA and for cells that were transfected with CRISPR/Cas system tethered with certain exonucleases via heterodimerizing peptide pairs; for example Cas9-P3 with combination of P4-EXOIII, but the band intensities, which is the criterion for indel mutation determination, are the same in all samples.

### Example 6: Modified CRISPR/Cas system tethered with certain exonucleases via heterodimerizing peptide pairs poses anticancer therapeutical properties

Modified CRISPR/Cas system tethered with certain exonucleases via heterodimerizing peptide pairs poses anticancer therapeutical properties when the cause of cancer is in genomic rearrangements. CML (chronic myelogenous leukemia) patients in all cases bear Philadelphia chromosome or *BCR-ABL* fusion gene (t9, 22; q34;q11) between chromosome 9 and 22. This chromosome has the causative role for coding a hybrid kinase, a tyrosine kinase signaling protein. Constitutive signaling of this kinase is causing cells uncontrollably to divide and proliferate. To determine the possible anti-CML therapeutic option, K562 cells (stably expressing firefly luciferase; K562-fLUC), which carry *BCR-ABL* fusion gene were electroporated with plasmid DNA of designated CRISPR/Cas9 constructs. gRNA with targeting sequence *GACCTGTCTTTTAGACAGGC* for leading Cas9 to *BCR-ABL* fusion gene was used. 72 hours after electroporation of K562-fLUC cells, D-luciferin (Xenogen) was added and bioluminescence was measured by using IVIS^{®} Lumina Series III (Perkin Elmer). Data were analyzed with Living Image^{®} 4.5.2 (Perkin Elmer).

To test the activity of modified CRISPR/Cas system tethered with certain exonucleases via heterodimerizing peptide pairs K562 cells were used to determine the activity of modified CRISPR/Cas system tethered with certain exonucleases via heterodimerizing peptide pairs. Cells were electroporated with constructs that express CRISPR/Cas system tethered with certain exonucleases via heterodimerizing peptide pairs; for example Cas9-P3 or Cas9-P4 or Cas9-N5 with combination of P4-EXOIII or P3-EXOIII or N6-EXOIII. For control, cells were electroporated with regular CRISPR/Cas9 system or were cooexpressed with CRISPR/Cas9 system and certain exonuclease (e.g. EXOIII). gRNA for human *BCR-ABL* fusion gene (targeting sequence *GACCTGTCTTTTAGACAGGC*) was used. 48 hours later genomic DNA from cells was isolated and genomic region around predicted DSB was PCR amplified. Next T7E1 assay was carried out. Presence and quantification of indel mutation was determined by Syber gold stained PAGE (polyacrylamide gel electrophoresis) gel analysis. Percentage of indel mutations was calculated by ImageJ software.

### Result:

Figure 9 shows bioluminescence of cell population of K562-fLUC cells after CRISPR/Cas constructs electroporation. Bioluminescence reduction demonstrates cell death due to genomic editing at *BCR-ABL* fusion gene genomic area. Drop of bioluminescent signal is seen in cells that are electroporated with regular CRISPR/Cas9 system, where only Cas9 and gRNA are expressed, compared to cells, electroporated with empty pcDNA3 vector. Additional drop of bioluminescence is observed when cooexpression of Cas9 and certain exonuclease (e.g. EXOIII) and gRNA is used. The biggest loss of bioluminescent signal due to the highest genome editing at *BCR-ABL* fusion gene genomic area and consequently cell death is observed in cells that were electroporated with CRISPR/Cas system tethered with certain exonucleases via heterodimerizing peptide pairs; for example Cas9-P3 or Cas9-P4 with combination of P4-EXOIII or P3-EXOIII and corresponding gRNA.

PAGE gel analysis of T7E1 treated samples from transfected cells with appropriate constructs in Figure 10A indicate that genome editing of human *BCR-ABL* fusion gene gene was highest among all tested samples when Cas9-N5 tethered to EXOIII via N6 peptide was used. A modest decrease in genome editing was observed when cells were transfected with constructs, expressing Cas9-P3 and P4-EXOIII or in cell samples with cooexpression of Cas9, gRNA targeting human *BCR-ABL* gene and certain exonucleases (e.g. EXOIII), but nevertheless in all samples genome editing was higher when only Cas9 and gRNA were used. No gene editing occurred when cells were electroporated with empty vector. Figure 10B graphically shows percentage of indel presence, which were determined with PAGE analysis, depicted in figure 10A.

## Claims

1. Combination comprising a first component (a) and a second component (b), wherein
a) the first component comprises a Cas9 endonuclease capable of inducing a double stranded DNA break, fused with a first protein or protein domain, and
b) the second component comprises an exonuclease selected from Exol, Exolll and mTREX2, fused to a second protein or protein domain,
wherein the first and the second components are capable of heterodimerizing with each other via interaction of the first protein or protein domain and the second protein or protein domain
wherein the first protein or protein domain and the second protein or protein domain are orthogonal protein partners P3 and P4 or N5 and N6, forming a coiled-coil structure, in particular a coiled-coil structure in parallel or antiparallel orientation.

2. The combination according to claim 1, wherein heterodimerization of the first protein or protein domain and the second protein or domain is inducible via a chemical or non-chemical signal.

3. The combination according to claim 2, wherein the chemical inducing heterodimerization is a small molecule such as rapalog, gibberellin or abscisic acid or any known small molecule.

4. The combination according to claim 2, wherein the non-chemical signal inducing heterodimerization is light with a predetermined wave length.

5. The combination according to any one of the preceding claims, further comprising a donor DNA molecule, which is a single-stranded or double-stranded DNA molecule, particularly a single-stranded DNA molecule, wherein the donor DNA molecule may carry a desired mutation.

6. A nucleic acid molecule, or a combination of nucleic acid molecules, comprising a first nucleic acid sequence encoding the first component (a) and a second nucleic acid sequence encoding the second component (b) of the combination according to any one of claims 1-5.

7. A vector comprising the nucleic acid molecule(s) according to claim 6.

8. A cell or organism comprising the combination according to any one of claims 1-5, the nucleic acid molecule(s) according to claim 6 or the vector according to claim 7, provided that the cell is not a human germ line cell.

9. A combination according to any one of claims 1-5, a nucleic acid molecule according to claim 6, a vector according to claim 7, or a cell or organism according to claim 8, for use in medicine.

10. The combination, nucleic acid molecule, vector, cell or organism for the use according to claim 9, in a method comprising genome editing in a eukaryotic target cell or in a eukaryotic target organism.

11. The combination, nucleic acid molecule, vector, cell or organism for the use according to claim 9 or 10, wherein the target cell is a vertebrate target cell, in particular a mammalian target cell, preferably a human target cell, for example a stem cell including an induced or embryonic pluripotent stem cell of an eukaryotic target organism, particularly a human induced or embryonic pluripotent stem cell, provided that the cell is not a human germ line cell, or wherein the target organism is a mammalian target organism, particularly a human.

12. The combination, nucleic acid molecule, vector, cell or organism for the use according to claim 10 or 11 when dependent on claim 10, wherein the genome editing comprises introducing a donor DNA molecule optionally carrying a desired mutation, which is a single-stranded or double-stranded DNA molecule, particularly a single-stranded DNA molecule, into the target cell or target organism.

13. An in vitro method for editing the genome of a eukaryotic target cell comprising introducing a combination as defined in any one of claims 1 - 5, a nucleic acid molecule according to claim 6, or a vector according to claim 7 into the target cell, provided that the cell is not a human germ line cell.

14. In vitro use of a combination as defined in any one of claims 1-5, a nucleic acid molecule according to claim 6, or a vector according to claim 7, for genome editing in a eukaryotic target cell, particularly in a mammalian target cell, more particularly in a human target cell, provided that the cell is not a human germ line cell.

## Patentansprüche

1. Kombination, umfassend eine erste Komponente (a) und eine zweite Komponente (b), wobei
a) die erste Komponente eine Cas9-Endonuklease umfasst, die in der Lage ist, einen Doppelstrang-DNA-Bruch zu induzieren, fusioniert mit einem ersten Protein oder einer ersten Proteindomäne, und
b) die zweite Komponente eine Exonuklease, ausgewählt aus Exol, Exolll und mTREX2 umfasst, fusioniert mit einem zweiten Protein oder einer zweiten Proteindomäne,
wobei die erste und die zweite Komponente in der Lage sind, über eine Wechselwirkung des ersten Proteins oder der ersten Proteindomäne und des zweiten Proteins oder der zweiten Proteindomäne miteinander zu heterodimerisieren,
wobei das erste Protein oder die erste Proteindomäne und das zweite Protein oder die zweite Proteindomäne orthogonale Proteinpartner P3 und P4 oder N5 und N6 sind, die eine Coiled-Coil-Struktur bilden, insbesondere eine Coiled-Coil-Struktur in paralleler oder antiparalleler Ausrichtung.

2. Kombination nach Anspruch 1, wobei die Heterodimerisierung des ersten Proteins oder der ersten Proteindomäne und des zweiten Proteins oder der zweiten Domäne durch ein chemisches oder nicht-chemisches Signal induzierbar ist.

3. Kombination nach Anspruch 2, wobei die Chemikalie, die die Heterodimerisierung induziert, ein kleines Molekül wie Rapalog, Gibberellin oder Abscisinsäure oder irgendein anderes bekanntes kleines Molekül ist.

4. Kombination nach Anspruch 2, wobei das nicht-chemische Signal, das die Heterodimerisierung induziert, Licht mit einer vorgegebenen Wellenlänge ist.

5. Kombination nach einem der vorhergehenden Ansprüche, die ferner ein Donor-DNA-Molekül umfasst, das ein einzelsträngiges oder doppelsträngiges DNA-Molekül, insbesondere ein einzelsträngiges DNA-Molekül, ist, wobei das Donor-DNA-Molekül eine gewünschte Mutation tragen kann.

6. Nukleinsäuremolekül oder Kombination von Nukleinsäuremolekülen, umfassend eine erste Nukleinsäuresequenz, die für die erste Komponente (a) kodiert, und eine zweite Nukleinsäuresequenz, die für die zweite Komponente (b) der Kombination nach einem der Ansprüche 1 - 5 kodiert.

7. Vektor, umfassend das Nukleinsäuremolekül/die Nukleinsäuremoleküle nach Anspruch 6.

8. Zelle oder Organismus, umfassend die Kombination nach einem der Ansprüche 1 - 5, das/die Nukleinsäuremolekül(e) nach Anspruch 6 oder den Vektor nach Anspruch 7, mit der Maßgabe, dass die Zelle keine menschliche Keimbahnzelle ist.

9. Kombination nach einem der Ansprüche 1 bis 5, Nukleinsäuremolekül nach Anspruch 6, Vektor nach Anspruch 7 oder Zelle oder Organismus nach Anspruch 8 zur Verwendung in der Medizin.

10. Kombination, Nukleinsäuremolekül, Vektor, Zelle oder Organismus zur Verwendung nach Anspruch 9 in einem Verfahren, das Genome-Editing in einer eukaryotischen Zielzelle oder in einem eukaryotischen Zielorganismus umfasst.

11. Kombination, Nukleinsäuremolekül, Vektor, Zelle oder Organismus zur Verwendung nach Anspruch 9 oder 10, wobei die Zielzelle eine Wirbeltier-Zielzelle ist, insbesondere eine Säuger-Zielzelle, vorzugsweise eine menschliche Zielzelle, beispielsweise eine Stammzelle, einschließlich einer induzierten oder embryonalen pluripotenten Stammzelle eines eukaryotischen Zielorganismus, insbesondere eine menschliche induzierte oder embryonale pluripotente Stammzelle, mit der Maßgabe, dass die Zelle keine menschliche Keimbahnzelle ist, oder wobei der Zielorganismus ein Säuger-Zielorganismus, insbesondere ein Mensch, ist.

12. Kombination, Nukleinsäuremolekül, Vektor, Zelle oder Organismus zur Verwendung nach Anspruch 10 oder 11, wenn sie von Anspruch 10 abhängen, wobei das Genom-Editing das Einführen eines Donor-DNA-Moleküls, das gegebenenfalls eine gewünschte Mutation trägt und ein einzelsträngiges oder doppelsträngiges DNA-Molekül, insbesondere ein einzelsträngiges DNA-Molekül, ist, in die Zielzelle oder den Zielorganismus umfasst.

13. In-vitro-Verfahren zum Editieren des Genoms einer eukaryotischen Zielzelle, umfassend das Einführen einer Kombination wie in einem der Ansprüche 1- 5 definiert, eines Nukleinsäuremoleküls nach Anspruch 6 oder eines Vektors nach Anspruch 7 in die Zielzelle, mit der Maßgabe, dass die Zelle keine menschliche Keimbahnzelle ist.

14. In-vitro-Verwendung einer Kombination wie in einem der Ansprüche 1 - 5 definiert, eines Nukleinsäuremoleküls nach Anspruch 6 oder eines Vektors nach Anspruch 7 zum Genome-Editing in einer eukaryotischen Zielzelle, insbesondere in einer Säuger-Zielzelle, spezieller in einer menschlichen Zielzelle, mit der Maßgabe, dass die Zelle keine menschliche Keimbahnzelle ist.

## Revendications

1. Combinaison comprenant un premier composant (a) et un second composant (b), où
a) le premier composant comprend une endonucléase Cas9 capable d'induire une cassure d'ADN double brin, fusionnée à une première protéine ou à un premier domaine protéique, et
b) le second composant comprend une exonucléase choisie parmi ExoI, ExoIII et mTREX2, fusionnée à une seconde protéine ou à un second domaine protéique,
où les premier et second composants sont capables de s'hétérodimériser l'un avec l'autre par interaction de la première protéine ou du premier domaine protéique et de la seconde protéine ou du second domaine protéique.
où la première protéine ou le premier domaine protéique et la seconde protéine ou le second domaine protéique sont des partenaires protéiques orthogonaux P3 et P4 ou N5 et N6, formant une structure de superhélice, en particulier une structure de superhélice dans une orientation parallèle ou antiparallèle.

2. Combinaison selon la revendication 1, où l'hétérodimérisation de la première protéine ou du premier domaine protéique et de la seconde protéine ou du second domaine est inductible par un signal chimique ou non chimique.

3. Combinaison selon la revendication 2, où le produit chimique induisant l'hétérodimérisation est une petite molécule telle que rapalogue, gibbérelline ou acide abscisique, ou toute autre petite molécule connue.

4. Combinaison selon la revendication 2, où le signal non chimique induisant l'hétérodimérisation est une lumière ayant une longueur d'onde prédéterminée.

5. Combinaison selon l'une quelconque des revendications précédentes, comprenant en outre une molécule d'ADN donneur, qui est une molécule d'ADN simple brin ou double brin, en particulier une molécule d'ADN simple brin, où la molécule d'ADN donneur peut porter une mutation souhaitée.

6. Molécule d'acide nucléique, ou combinaison de molécules d'acide nucléique, comprenant une première séquence d'acide nucléique codant le premier composant (a) et une seconde séquence d'acide nucléique codant le second composant (b) de la combinaison selon l'une quelconque des revendications 1 à 5.

7. Vecteur comprenant la ou les molécules d'acide nucléique selon la revendication 6.

8. Cellule ou organisme comprenant la combinaison selon l'une quelconque des revendications 1 à 5, la ou les molécules d'acide nucléique selon la revendication 6 ou le vecteur selon la revendication 7, à condition que la cellule ne soit pas une cellule germinale humaine.

9. Combinaison selon l'une quelconque des revendications 1 à 5, molécule d'acide nucléique selon la revendication 6, vecteur selon la revendication 7, ou cellule ou organisme selon la revendication 8, pour une utilisation en médecine.

10. Combinaison, molécule d'acide nucléique, vecteur, cellule ou organisme pour l'utilisation selon la revendication 9, dans un procédé comprenant l'édition du génome dans une cellule cible eucaryote ou dans un organisme cible eucaryote.

11. Combinaison, molécule d'acide nucléique, vecteur, cellule ou organisme pour l'utilisation selon la revendication 9 ou 10, où la cellule cible est une cellule cible de vertébré, en particulier une cellule cible de mammifère, de préférence une cellule cible humaine, par exemple une cellule souche incluant une cellule souche pluripotente induite ou embryonnaire d'un organisme cible eucaryote, en particulier une cellule souche pluripotente induite ou embryonnaire humaine, à condition que la cellule ne soit pas une cellule germinale humaine, ou où l'organisme cible est un organisme cible de mammifère, en particulier un humain.

12. Combinaison, molécule d'acide nucléique, vecteur, cellule ou organisme pour l'utilisation selon la revendication 10 ou 11 lorsqu'elle dépend de la revendication 10 où l'édition du génome comprend l'introduction d'une molécule d'ADN donneur portant éventuellement une mutation souhaitée, qui est une molécule d'ADN simple brin ou double brin, en particulier une molécule d'ADN simple brin, dans la cellule cible ou l'organisme cible.

13. Procédé in vitro d'édition du génome d'une cellule cible eucaryote comprenant l'introduction d'une combinaison telle que définie dans l'une quelconque des revendications 1 à 5, d'une molécule d'acide nucléique selon la revendication 6 ou d'un vecteur selon la revendication 7 dans la cellule cible, à condition que la cellule ne soit pas une cellule germinale humaine.

14. Utilisation in vitro d'une combinaison telle que définie dans l'une quelconque des revendications 1 à 5, d'une molécule d'acide nucléique selon la revendication 6 ou d'un vecteur selon la revendication 7, pour l'édition du génome dans une cellule cible eucaryote, en particulier dans une cellule cible de mammifère, plus particulièrement dans une cellule cible humaine, à condition que la cellule ne soit pas une cellule germinale humaine.


### SEQUENCE LISTING

<110> National Institute of Chemistry
<120> coiled-coil mediated tethering of CRISPR/CAS and exonucleases for enhanced genome editing
<130> 69116P EP
<160> 18
<170> PatentIn version 3.5
<210> 1
   <211> 4308
   <212> DNA
   <213> Artificial Sequence
<220>
   <223> Cas9-P4
<400> 1
   atggccccaa agaagaagcg gaaggtcggt atccacggag tcccagcagc cgacaagaag      60
   tacagcatcg gcctggacat cggcaccaac tctgtgggct gggccgtgat caccgacgag     120
   tacaaggtgc ccagcaagaa attcaaggtg ctgggcaaca ccgaccggca cagcatcaag     180
   aagaacctga tcggagccct gctgttcgac agcggcgaaa cagccgaggc cacccggctg     240
   aagagaaccg ccagaagaag atacaccaga cggaagaacc ggatctgcta tctgcaagag     300
   atcttcagca acgagatggc caaggtggac gacagcttct tccacagact ggaagagtcc     360
   ttcctggtgg aagaggataa gaagcacgag cggcacccca tcttcggcaa catcgtggac     420
   gaggtggcct accacgagaa gtaccccacc atctaccacc tgagaaagaa actggtggac     480
   agcaccgaca aggccgacct gcggctgatc tatctggccc tggcccacat gatcaagttc     540
   cggggccact tcctgatcga gggcgacctg aaccccgaca acagcgacgt ggacaagctg     600
   ttcatccagc tggtgcagac ctacaaccag ctgttcgagg aaaaccccat caacgccagc     660
   ggcgtggacg ccaaggccat cctgtctgcc agactgagca agagcagacg gctggaaaat     720
   ctgatcgccc agctgcccgg cgagaagaag aatggcctgt tcggaaacct gattgccctg     780
   agcctgggcc tgacccccaa cttcaagagc aacttcgacc tggccgagga tgccaaactg     840
   cagctgagca aggacaccta cgacgacgac ctggacaacc tgctggccca gatcggcgac     900
   cagtacgccg acctgtttct ggccgccaag aacctgtccg acgccatcct gctgagcgac     960
   atcctgagag tgaacaccga gatcaccaag gcccccctga gcgcctctat gatcaagaga 1020
   tacgacgagc accaccagga cctgaccctg ctgaaagctc tcgtgcggca gcagctgcct 1080
   gagaagtaca aagagatttt cttcgaccag agcaagaacg gctacgccgg ctacattgac 1140
   ggcggagcca gccaggaaga gttctacaag ttcatcaagc ccatcctgga aaagatggac 1200
   ggcaccgagg aactgctcgt gaagctgaac agagaggacc tgctgcggaa gcagcggacc 1260
   ttcgacaacg gcagcatccc ccaccagatc cacctgggag agctgcacgc cattctgcgg 1320
   cggcaggaag atttttaccc attcctgaag gacaaccggg aaaagatcga gaagatcctg 1380
   accttccgca tcccctacta cgtgggccct ctggccaggg gaaacagcag attcgcctgg 1440
   atgaccagaa agagcgagga aaccatcacc ccctggaact tcgaggaagt ggtggacaag 1500
   ggcgcttccg cccagagctt catcgagcgg atgaccaact tcgataagaa cctgcccaac 1560
   gagaaggtgc tgcccaagca cagcctgctg tacgagtact tcaccgtgta taacgagctg 1620
   accaaagtga aatacgtgac cgagggaatg agaaagcccg ccttcctgag cggcgagcag 1680
   aaaaaggcca tcgtggacct gctgttcaag accaaccgga aagtgaccgt gaagcagctg 1740
   aaagaggact acttcaagaa aatcgagtgc ttcgactccg tggaaatctc cggcgtggaa 1800
   gatcggttca acgcctccct gggcacatac cacgatctgc tgaaaattat caaggacaag 1860
   gacttcctgg acaatgagga aaacgaggac attctggaag atatcgtgct gaccctgaca 1920
   ctgtttgagg acagagagat gatcgaggaa cggctgaaaa cctatgccca cctgttcgac 1980
   gacaaagtga tgaagcagct gaagcggcgg agatacaccg gctggggcag gctgagccgg 2040
   aagctgatca acggcatccg ggacaagcag tccggcaaga caatcctgga tttcctgaag 2100
   tccgacggct tcgccaacag aaacttcatg cagctgatcc acgacgacag cctgaccttt 2160
   aaagaggaca tccagaaagc ccaggtgtcc ggccagggcg atagcctgca cgagcacatt 2220
   gccaatctgg ccggcagccc cgccattaag aagggcatcc tgcagacagt gaaggtggtg 2280
   gacgagctcg tgaaagtgat gggccggcac aagcccgaga acatcgtgat cgaaatggcc 2340
   agagagaacc agaccaccca gaagggacag aagaacagcc gcgagagaat gaagcggatc 2400
   gaagagggca tcaaagagct gggcagccag atcctgaaag aacaccccgt ggaaaacacc 2460
   cagctgcaga acgagaagct gtacctgtac tacctgcaga atgggcggga tatgtacgtg 2520
   gaccaggaac tggacatcaa ccggctgtcc gactacgatg tggaccatat cgtgcctcag 2580
   agctttctga aggacgactc catcgacaac aaggtgctga ccagaagcga caagaaccgg 2640
   ggcaagagcg acaacgtgcc ctccgaagag gtcgtgaaga agatgaagaa ctactggcgg    2700
   cagctgctga acgccaagct gattacccag agaaagttcg acaatctgac caaggccgag    2760
   agaggcggcc tgagcgaact ggataaggcc ggcttcatca agagacagct ggtggaaacc    2820
   cggcagatca caaagcacgt ggcacagatc ctggactccc ggatgaacac taagtacgac    2880
   gagaatgaca agctgatccg ggaagtgaaa gtgatcaccc tgaagtccaa gctggtgtcc    2940
   gatttccgga aggatttcca gttttacaaa gtgcgcgaga tcaacaacta ccaccacgcc    3000
   cacgacgcct acctgaacgc cgtcgtggga accgccctga tcaaaaagta ccctaagctg    3060
   gaaagcgagt tcgtgtacgg cgactacaag gtgtacgacg tgcggaagat gatcgccaag    3120
   agcgagcagg aaatcggcaa ggctaccgcc aagtacttct tctacagcaa catcatgaac    3180
   tttttcaaga ccgagattac cctggccaac ggcgagatcc ggaagcggcc tctgatcgag    3240
   acaaacggcg aaaccgggga gatcgtgtgg gataagggcc gggattttgc caccgtgcgg    3300
   aaagtgctga gcatgcccca agtgaatatc gtgaaaaaga ccgaggtgca gacaggcggc    3360
   ttcagcaaag agtctatcct gcccaagagg aacagcgata agctgatcgc cagaaagaag    3420
   gactgggacc ctaagaagta cggcggcttc gacagcccca ccgtggccta ttctgtgctg    3480
   gtggtggcca aagtggaaaa gggcaagtcc aagaaactga agagtgtgaa agagctgctg    3540
   gggatcacca tcatggaaag aagcagcttc gagaagaatc ccatcgactt tctggaagcc    3600
   aagggctaca aagaagtgaa aaaggacctg atcatcaagc tgcctaagta ctccctgttc    3660
   gagctggaaa acggccggaa gagaatgctg gcctctgccg gcgaactgca gaagggaaac    3720
   gaactggccc tgccctccaa atatgtgaac ttcctgtacc tggccagcca ctatgagaag    3780
   ctgaagggct cccccgagga taatgagcag aaacagctgt ttgtggaaca gcacaagcac    3840
   tacctggacg agatcatcga gcagatcagc gagttctcca agagagtgat cctggccgac    3900
   gctaatctgg acaaagtgct gtccgcctac aacaagcacc gggataagcc catcagagag    3960
   caggccgaga atatcatcca cctgtttacc ctgaccaatc tgggagcccc tgccgccttc    4020
   aagtactttg acaccaccat cgaccggaag aggtacacca gcaccaaaga ggtgctggac    4080
   gccaccctga tccaccagag catcaccggc ctgtacgaga cacggatcga cctgtctcag    4140
   ctgggaggcg acgatccaaa aaagaagaga aaggtaggcg gctctggcgg cggctccgga    4200
   ggctctagcc cggaagataa aattgctcag ctgaaacaaa aaatccaagc gctgaaacag    4260
   gaaaaccagc agctggaaga ggaaaacgcc gcactggaat atggttaa                 4308
<210> 2
   <211> 4308
   <212> DNA
   <213> Artificial sequence
<220>
   <223> Cas9-P3
<400> 2
   atggccccaa agaagaagcg gaaggtcggt atccacggag tcccagcagc cgacaagaag 60
   tacagcatcg gcctggacat cggcaccaac tctgtgggct gggccgtgat caccgacgag 120
   tacaaggtgc ccagcaagaa attcaaggtg ctgggcaaca ccgaccggca cagcatcaag 180
   aagaacctga tcggagccct gctgttcgac agcggcgaaa cagccgaggc cacccggctg 240
   aagagaaccg ccagaagaag atacaccaga cggaagaacc ggatctgcta tctgcaagag 300
   atcttcagca acgagatggc caaggtggac gacagcttct tccacagact ggaagagtcc 360
   ttcctggtgg aagaggataa gaagcacgag cggcacccca tcttcggcaa catcgtggac 420
   gaggtggcct accacgagaa gtaccccacc atctaccacc tgagaaagaa actggtggac 480
   agcaccgaca aggccgacct gcggctgatc tatctggccc tggcccacat gatcaagttc 540
   cggggccact tcctgatcga gggcgacctg aaccccgaca acagcgacgt ggacaagctg 600
   ttcatccagc tggtgcagac ctacaaccag ctgttcgagg aaaaccccat caacgccagc 660
   ggcgtggacg ccaaggccat cctgtctgcc agactgagca agagcagacg gctggaaaat 720
   ctgatcgccc agctgcccgg cgagaagaag aatggcctgt tcggaaacct gattgccctg 780
   agcctgggcc tgacccccaa cttcaagagc aacttcgacc tggccgagga tgccaaactg 840
   cagctgagca aggacaccta cgacgacgac ctggacaacc tgctggccca gatcggcgac 900
   cagtacgccg acctgtttct ggccgccaag aacctgtccg acgccatcct gctgagcgac 960
   atcctgagag tgaacaccga gatcaccaag gcccccctga gcgcctctat gatcaagaga 1020
   tacgacgagc accaccagga cctgaccctg ctgaaagctc tcgtgcggca gcagctgcct 1080
   gagaagtaca aagagatttt cttcgaccag agcaagaacg gctacgccgg ctacattgac 1140
   ggcggagcca gccaggaaga gttctacaag ttcatcaagc ccatcctgga aaagatggac 1200
   ggcaccgagg aactgctcgt gaagctgaac agagaggacc tgctgcggaa gcagcggacc 1260
   ttcgacaacg gcagcatccc ccaccagatc cacctgggag agctgcacgc cattctgcgg 1320
   cggcaggaag atttttaccc attcctgaag gacaaccggg aaaagatcga gaagatcctg 1380
   accttccgca tcccctacta cgtgggccct ctggccaggg gaaacagcag attcgcctgg      1440
   atgaccagaa agagcgagga aaccatcacc ccctggaact tcgaggaagt ggtggacaag      1500
   ggcgcttccg cccagagctt catcgagcgg atgaccaact tcgataagaa cctgcccaac      1560
   gagaaggtgc tgcccaagca cagcctgctg tacgagtact tcaccgtgta taacgagctg      1620
   accaaagtga aatacgtgac cgagggaatg agaaagcccg ccttcctgag cggcgagcag      1680
   aaaaaggcca tcgtggacct gctgttcaag accaaccgga aagtgaccgt gaagcagctg      1740
   aaagaggact acttcaagaa aatcgagtgc ttcgactccg tggaaatctc cggcgtggaa      1800
   gatcggttca acgcctccct gggcacatac cacgatctgc tgaaaattat caaggacaag      1860
   gacttcctgg acaatgagga aaacgaggac attctggaag atatcgtgct gaccctgaca      1920
   ctgtttgagg acagagagat gatcgaggaa cggctgaaaa cctatgccca cctgttcgac      1980
   gacaaagtga tgaagcagct gaagcggcgg agatacaccg gctggggcag gctgagccgg      2040
   aagctgatca acggcatccg ggacaagcag tccggcaaga caatcctgga tttcctgaag      2100
   tccgacggct tcgccaacag aaacttcatg cagctgatcc acgacgacag cctgaccttt      2160
   aaagaggaca tccagaaagc ccaggtgtcc ggccagggcg atagcctgca cgagcacatt      2220
   gccaatctgg ccggcagccc cgccattaag aagggcatcc tgcagacagt gaaggtggtg      2280
   gacgagctcg tgaaagtgat gggccggcac aagcccgaga acatcgtgat cgaaatggcc      2340
   agagagaacc agaccaccca gaagggacag aagaacagcc gcgagagaat gaagcggatc      2400
   gaagagggca tcaaagagct gggcagccag atcctgaaag aacaccccgt ggaaaacacc      2460
   cagctgcaga acgagaagct gtacctgtac tacctgcaga atgggcggga tatgtacgtg      2520
   gaccaggaac tggacatcaa ccggctgtcc gactacgatg tggaccatat cgtgcctcag      2580
   agctttctga aggacgactc catcgacaac aaggtgctga ccagaagcga caagaaccgg      2640
   ggcaagagcg acaacgtgcc ctccgaagag gtcgtgaaga agatgaagaa ctactggcgg      2700
   cagctgctga acgccaagct gattacccag agaaagttcg acaatctgac caaggccgag      2760
   agaggcggcc tgagcgaact ggataaggcc ggcttcatca agagacagct ggtggaaacc      2820
   cggcagatca caaagcacgt ggcacagatc ctggactccc ggatgaacac taagtacgac      2880
   gagaatgaca agctgatccg ggaagtgaaa gtgatcaccc tgaagtccaa gctggtgtcc      2940
   gatttccgga aggatttcca gttttacaaa gtgcgcgaga tcaacaacta ccaccacgcc      3000
   cacgacgcct acctgaacgc cgtcgtggga accgccctga tcaaaaagta ccctaagctg      3060
   gaaagcgagt tcgtgtacgg cgactacaag gtgtacgacg tgcggaagat gatcgccaag 3120
   agcgagcagg aaatcggcaa ggctaccgcc aagtacttct tctacagcaa catcatgaac 3180
   tttttcaaga ccgagattac cctggccaac ggcgagatcc ggaagcggcc tctgatcgag 3240
   acaaacggcg aaaccgggga gatcgtgtgg gataagggcc gggattttgc caccgtgcgg 3300
   aaagtgctga gcatgcccca agtgaatatc gtgaaaaaga ccgaggtgca gacaggcggc 3360
   ttcagcaaag agtctatcct gcccaagagg aacagcgata agctgatcgc cagaaagaag 3420
   gactgggacc ctaagaagta cggcggcttc gacagcccca ccgtggccta ttctgtgctg 3480
   gtggtggcca aagtggaaaa gggcaagtcc aagaaactga agagtgtgaa agagctgctg 3540
   gggatcacca tcatggaaag aagcagcttc gagaagaatc ccatcgactt tctggaagcc 3600
   aagggctaca aagaagtgaa aaaggacctg atcatcaagc tgcctaagta ctccctgttc 3660
   gagctggaaa acggccggaa gagaatgctg gcctctgccg gcgaactgca gaagggaaac 3720
   gaactggccc tgccctccaa atatgtgaac ttcctgtacc tggccagcca ctatgagaag 3780
   ctgaagggct cccccgagga taatgagcag aaacagctgt ttgtggaaca gcacaagcac 3840
   tacctggacg agatcatcga gcagatcagc gagttctcca agagagtgat cctggccgac 3900
   gctaatctgg acaaagtgct gtccgcctac aacaagcacc gggataagcc catcagagag 3960
   caggccgaga atatcatcca cctgtttacc ctgaccaatc tgggagcccc tgccgccttc 4020
   aagtactttg acaccaccat cgaccggaag aggtacacca gcaccaaaga ggtgctggac 4080
   gccaccctga tccaccagag catcaccggc ctgtacgaga cacggatcga cctgtctcag 4140
   ctgggaggcg acgatccaaa aaagaagaga aaggtaggcg gctctggcgg cggctccgga 4200
   ggctcttccc cggaagatga gatccagcaa ctggaagaag aaatcgctca gctggaacag 4260
   aaaaacgcag cgctgaaaga gaaaaaccag gcgctgaaat acggttaa 4308
<210> 3
   <211> 4293
   <212> DNA
   <213> Artificial sequence
<220>
   <223> Cas9-N5
<400> 3
   atggccccaa agaagaagcg gaaggtcggt atccacggag tcccagcagc cgacaagaag 60
   tacagcatcg gcctggacat cggcaccaac tctgtgggct gggccgtgat caccgacgag 120
   tacaaggtgc ccagcaagaa attcaaggtg ctgggcaaca ccgaccggca cagcatcaag 180
   aagaacctga tcggagccct gctgttcgac agcggcgaaa cagccgaggc cacccggctg 240
   aagagaaccg ccagaagaag atacaccaga cggaagaacc ggatctgcta tctgcaagag 300
   atcttcagca acgagatggc caaggtggac gacagcttct tccacagact ggaagagtcc 360
   ttcctggtgg aagaggataa gaagcacgag cggcacccca tcttcggcaa catcgtggac 420
   gaggtggcct accacgagaa gtaccccacc atctaccacc tgagaaagaa actggtggac 480
   agcaccgaca aggccgacct gcggctgatc tatctggccc tggcccacat gatcaagttc 540
   cggggccact tcctgatcga gggcgacctg aaccccgaca acagcgacgt ggacaagctg 600
   ttcatccagc tggtgcagac ctacaaccag ctgttcgagg aaaaccccat caacgccagc 660
   ggcgtggacg ccaaggccat cctgtctgcc agactgagca agagcagacg gctggaaaat 720
   ctgatcgccc agctgcccgg cgagaagaag aatggcctgt tcggaaacct gattgccctg 780
   agcctgggcc tgacccccaa cttcaagagc aacttcgacc tggccgagga tgccaaactg 840
   cagctgagca aggacaccta cgacgacgac ctggacaacc tgctggccca gatcggcgac 900
   cagtacgccg acctgtttct ggccgccaag aacctgtccg acgccatcct gctgagcgac 960
   atcctgagag tgaacaccga gatcaccaag gcccccctga gcgcctctat gatcaagaga 1020
   tacgacgagc accaccagga cctgaccctg ctgaaagctc tcgtgcggca gcagctgcct 1080
   gagaagtaca aagagatttt cttcgaccag agcaagaacg gctacgccgg ctacattgac 1140
   ggcggagcca gccaggaaga gttctacaag ttcatcaagc ccatcctgga aaagatggac 1200
   ggcaccgagg aactgctcgt gaagctgaac agagaggacc tgctgcggaa gcagcggacc 1260
   ttcgacaacg gcagcatccc ccaccagatc cacctgggag agctgcacgc cattctgcgg 1320
   cggcaggaag atttttaccc attcctgaag gacaaccggg aaaagatcga gaagatcctg 1380
   accttccgca tcccctacta cgtgggccct ctggccaggg gaaacagcag attcgcctgg 1440
   atgaccagaa agagcgagga aaccatcacc ccctggaact tcgaggaagt ggtggacaag 1500
   ggcgcttccg cccagagctt catcgagcgg atgaccaact tcgataagaa cctgcccaac 1560
   gagaaggtgc tgcccaagca cagcctgctg tacgagtact tcaccgtgta taacgagctg 1620
   accaaagtga aatacgtgac cgagggaatg agaaagcccg ccttcctgag cggcgagcag 1680
   aaaaaggcca tcgtggacct gctgttcaag accaaccgga aagtgaccgt gaagcagctg 1740
   aaagaggact acttcaagaa aatcgagtgc ttcgactccg tggaaatctc cggcgtggaa 1800
   gatcggttca acgcctccct gggcacatac cacgatctgc tgaaaattat caaggacaag 1860
   gacttcctgg acaatgagga aaacgaggac attctggaag atatcgtgct gaccctgaca 1920
   ctgtttgagg acagagagat gatcgaggaa cggctgaaaa cctatgccca cctgttcgac 1980
   gacaaagtga tgaagcagct gaagcggcgg agatacaccg gctggggcag gctgagccgg 2040
   aagctgatca acggcatccg ggacaagcag tccggcaaga caatcctgga tttcctgaag 2100
   tccgacggct tcgccaacag aaacttcatg cagctgatcc acgacgacag cctgaccttt 2160
   aaagaggaca tccagaaagc ccaggtgtcc ggccagggcg atagcctgca cgagcacatt 2220
   gccaatctgg ccggcagccc cgccattaag aagggcatcc tgcagacagt gaaggtggtg 2280
   gacgagctcg tgaaagtgat gggccggcac aagcccgaga acatcgtgat cgaaatggcc 2340
   agagagaacc agaccaccca gaagggacag aagaacagcc gcgagagaat gaagcggatc 2400
   gaagagggca tcaaagagct gggcagccag atcctgaaag aacaccccgt ggaaaacacc 2460
   cagctgcaga acgagaagct gtacctgtac tacctgcaga atgggcggga tatgtacgtg 2520
   gaccaggaac tggacatcaa ccggctgtcc gactacgatg tggaccatat cgtgcctcag 2580
   agctttctga aggacgactc catcgacaac aaggtgctga ccagaagcga caagaaccgg 2640
   ggcaagagcg acaacgtgcc ctccgaagag gtcgtgaaga agatgaagaa ctactggcgg 2700
   cagctgctga acgccaagct gattacccag agaaagttcg acaatctgac caaggccgag 2760
   agaggcggcc tgagcgaact ggataaggcc ggcttcatca agagacagct ggtggaaacc 2820
   cggcagatca caaagcacgt ggcacagatc ctggactccc ggatgaacac taagtacgac 2880
   gagaatgaca agctgatccg ggaagtgaaa gtgatcaccc tgaagtccaa gctggtgtcc 2940
   gatttccgga aggatttcca gttttacaaa gtgcgcgaga tcaacaacta ccaccacgcc 3000
   cacgacgcct acctgaacgc cgtcgtggga accgccctga tcaaaaagta ccctaagctg 3060
   gaaagcgagt tcgtgtacgg cgactacaag gtgtacgacg tgcggaagat gatcgccaag 3120
   agcgagcagg aaatcggcaa ggctaccgcc aagtacttct tctacagcaa catcatgaac 3180
   tttttcaaga ccgagattac cctggccaac ggcgagatcc ggaagcggcc tctgatcgag 3240
   acaaacggcg aaaccgggga gatcgtgtgg gataagggcc gggattttgc caccgtgcgg 3300
   aaagtgctga gcatgcccca agtgaatatc gtgaaaaaga ccgaggtgca gacaggcggc 3360
   ttcagcaaag agtctatcct gcccaagagg aacagcgata agctgatcgc cagaaagaag 3420
   gactgggacc ctaagaagta cggcggcttc gacagcccca ccgtggccta ttctgtgctg 3480
   gtggtggcca aagtggaaaa gggcaagtcc aagaaactga agagtgtgaa agagctgctg 3540
   gggatcacca tcatggaaag aagcagcttc gagaagaatc ccatcgactt tctggaagcc 3600
   aagggctaca aagaagtgaa aaaggacctg atcatcaagc tgcctaagta ctccctgttc 3660
   gagctggaaa acggccggaa gagaatgctg gcctctgccg gcgaactgca gaagggaaac 3720
   gaactggccc tgccctccaa atatgtgaac ttcctgtacc tggccagcca ctatgagaag 3780
   ctgaagggct cccccgagga taatgagcag aaacagctgt ttgtggaaca gcacaagcac 3840
   tacctggacg agatcatcga gcagatcagc gagttctcca agagagtgat cctggccgac 3900
   gctaatctgg acaaagtgct gtccgcctac aacaagcacc gggataagcc catcagagag 3960
   caggccgaga atatcatcca cctgtttacc ctgaccaatc tgggagcccc tgccgccttc 4020
   aagtactttg acaccaccat cgaccggaag aggtacacca gcaccaaaga ggtgctggac 4080
   gccaccctga tccaccagag catcaccggc ctgtacgaga cacggatcga cctgtctcag 4140
   ctgggaggcg acgatccaaa aaagaagaga aaggtaggcg gctctggcgg cggctccgga 4200
   ggctctgaga tcgccgccct ggaggccaag atcgccgccc tgaaggccaa gaacgccgcc 4260
   ctgaaggccg agatcgccgc cctggaggcc taa 4293
<210> 4
   <211> 4293
   <212> DNA
   <213> Artificial sequence
<220>
   <223> Cas9-N6
<400> 4
   atggccccaa agaagaagcg gaaggtcggt atccacggag tcccagcagc cgacaagaag 60
   tacagcatcg gcctggacat cggcaccaac tctgtgggct gggccgtgat caccgacgag 120
   tacaaggtgc ccagcaagaa attcaaggtg ctgggcaaca ccgaccggca cagcatcaag 180
   aagaacctga tcggagccct gctgttcgac agcggcgaaa cagccgaggc cacccggctg 240
   aagagaaccg ccagaagaag atacaccaga cggaagaacc ggatctgcta tctgcaagag 300
   atcttcagca acgagatggc caaggtggac gacagcttct tccacagact ggaagagtcc 360
   ttcctggtgg aagaggataa gaagcacgag cggcacccca tcttcggcaa catcgtggac 420
   gaggtggcct accacgagaa gtaccccacc atctaccacc tgagaaagaa actggtggac 480
   agcaccgaca aggccgacct gcggctgatc tatctggccc tggcccacat gatcaagttc 540
   cggggccact tcctgatcga gggcgacctg aaccccgaca acagcgacgt ggacaagctg 600
   ttcatccagc tggtgcagac ctacaaccag ctgttcgagg aaaaccccat caacgccagc 660
   ggcgtggacg ccaaggccat cctgtctgcc agactgagca agagcagacg gctggaaaat 720
   ctgatcgccc agctgcccgg cgagaagaag aatggcctgt tcggaaacct gattgccctg 780
   agcctgggcc tgacccccaa cttcaagagc aacttcgacc tggccgagga tgccaaactg 840
   cagctgagca aggacaccta cgacgacgac ctggacaacc tgctggccca gatcggcgac 900
   cagtacgccg acctgtttct ggccgccaag aacctgtccg acgccatcct gctgagcgac 960
   atcctgagag tgaacaccga gatcaccaag gcccccctga gcgcctctat gatcaagaga 1020
   tacgacgagc accaccagga cctgaccctg ctgaaagctc tcgtgcggca gcagctgcct 1080
   gagaagtaca aagagatttt cttcgaccag agcaagaacg gctacgccgg ctacattgac 1140
   ggcggagcca gccaggaaga gttctacaag ttcatcaagc ccatcctgga aaagatggac 1200
   ggcaccgagg aactgctcgt gaagctgaac agagaggacc tgctgcggaa gcagcggacc 1260
   ttcgacaacg gcagcatccc ccaccagatc cacctgggag agctgcacgc cattctgcgg 1320
   cggcaggaag atttttaccc attcctgaag gacaaccggg aaaagatcga gaagatcctg 1380
   accttccgca tcccctacta cgtgggccct ctggccaggg gaaacagcag attcgcctgg 1440
   atgaccagaa agagcgagga aaccatcacc ccctggaact tcgaggaagt ggtggacaag 1500
   ggcgcttccg cccagagctt catcgagcgg atgaccaact tcgataagaa cctgcccaac 1560
   gagaaggtgc tgcccaagca cagcctgctg tacgagtact tcaccgtgta taacgagctg 1620
   accaaagtga aatacgtgac cgagggaatg agaaagcccg ccttcctgag cggcgagcag 1680
   aaaaaggcca tcgtggacct gctgttcaag accaaccgga aagtgaccgt gaagcagctg 1740
   aaagaggact acttcaagaa aatcgagtgc ttcgactccg tggaaatctc cggcgtggaa 1800
   gatcggttca acgcctccct gggcacatac cacgatctgc tgaaaattat caaggacaag 1860
   gacttcctgg acaatgagga aaacgaggac attctggaag atatcgtgct gaccctgaca 1920
   ctgtttgagg acagagagat gatcgaggaa cggctgaaaa cctatgccca cctgttcgac 1980
   gacaaagtga tgaagcagct gaagcggcgg agatacaccg gctggggcag gctgagccgg 2040
   aagctgatca acggcatccg ggacaagcag tccggcaaga caatcctgga tttcctgaag 2100
   tccgacggct tcgccaacag aaacttcatg cagctgatcc acgacgacag cctgaccttt 2160
   aaagaggaca tccagaaagc ccaggtgtcc ggccagggcg atagcctgca cgagcacatt 2220
   gccaatctgg ccggcagccc cgccattaag aagggcatcc tgcagacagt gaaggtggtg 2280
   gacgagctcg tgaaagtgat gggccggcac aagcccgaga acatcgtgat cgaaatggcc 2340
   agagagaacc agaccaccca gaagggacag aagaacagcc gcgagagaat gaagcggatc 2400
   gaagagggca tcaaagagct gggcagccag atcctgaaag aacaccccgt ggaaaacacc 2460
   cagctgcaga acgagaagct gtacctgtac tacctgcaga atgggcggga tatgtacgtg 2520
   gaccaggaac tggacatcaa ccggctgtcc gactacgatg tggaccatat cgtgcctcag 2580
   agctttctga aggacgactc catcgacaac aaggtgctga ccagaagcga caagaaccgg 2640
   ggcaagagcg acaacgtgcc ctccgaagag gtcgtgaaga agatgaagaa ctactggcgg 2700
   cagctgctga acgccaagct gattacccag agaaagttcg acaatctgac caaggccgag 2760
   agaggcggcc tgagcgaact ggataaggcc ggcttcatca agagacagct ggtggaaacc 2820
   cggcagatca caaagcacgt ggcacagatc ctggactccc ggatgaacac taagtacgac 2880
   gagaatgaca agctgatccg ggaagtgaaa gtgatcaccc tgaagtccaa gctggtgtcc 2940
   gatttccgga aggatttcca gttttacaaa gtgcgcgaga tcaacaacta ccaccacgcc 3000
   cacgacgcct acctgaacgc cgtcgtggga accgccctga tcaaaaagta ccctaagctg 3060
   gaaagcgagt tcgtgtacgg cgactacaag gtgtacgacg tgcggaagat gatcgccaag 3120
   agcgagcagg aaatcggcaa ggctaccgcc aagtacttct tctacagcaa catcatgaac 3180
   tttttcaaga ccgagattac cctggccaac ggcgagatcc ggaagcggcc tctgatcgag 3240
   acaaacggcg aaaccgggga gatcgtgtgg gataagggcc gggattttgc caccgtgcgg 3300
   aaagtgctga gcatgcccca agtgaatatc gtgaaaaaga ccgaggtgca gacaggcggc 3360
   ttcagcaaag agtctatcct gcccaagagg aacagcgata agctgatcgc cagaaagaag 3420
   gactgggacc ctaagaagta cggcggcttc gacagcccca ccgtggccta ttctgtgctg 3480
   gtggtggcca aagtggaaaa gggcaagtcc aagaaactga agagtgtgaa agagctgctg 3540
   gggatcacca tcatggaaag aagcagcttc gagaagaatc ccatcgactt tctggaagcc 3600
   aagggctaca aagaagtgaa aaaggacctg atcatcaagc tgcctaagta ctccctgttc 3660
   gagctggaaa acggccggaa gagaatgctg gcctctgccg gcgaactgca gaagggaaac 3720
   gaactggccc tgccctccaa atatgtgaac ttcctgtacc tggccagcca ctatgagaag 3780
   ctgaagggct cccccgagga taatgagcag aaacagctgt ttgtggaaca gcacaagcac 3840
   tacctggacg agatcatcga gcagatcagc gagttctcca agagagtgat cctggccgac 3900
   gctaatctgg acaaagtgct gtccgcctac aacaagcacc gggataagcc catcagagag 3960
   caggccgaga atatcatcca cctgtttacc ctgaccaatc tgggagcccc tgccgccttc 4020
   aagtactttg acaccaccat cgaccggaag aggtacacca gcaccaaaga ggtgctggac 4080
   gccaccctga tccaccagag catcaccggc ctgtacgaga cacggatcga cctgtctcag 4140
   ctgggaggcg acgatccaaa aaagaagaga aaggtaggcg gctctggcgg cggctccgga 4200
   ggctctaaga tcgccgccct gaaggccgag atcgccgccc tggaggccga gaacgccgcc 4260
   ctggaggcca agatcgccgc cctgaaggcc taa 4293
<210> 5
   <211> 4305
   <212> DNA
   <213> Artificial sequence
<220>
   <223> Cas9-P3s
<400> 5
   atggccccaa agaagaagcg gaaggtcggt atccacggag tcccagcagc cgacaagaag 60
   tacagcatcg gcctggacat cggcaccaac tctgtgggct gggccgtgat caccgacgag 120
   tacaaggtgc ccagcaagaa attcaaggtg ctgggcaaca ccgaccggca cagcatcaag 180
   aagaacctga tcggagccct gctgttcgac agcggcgaaa cagccgaggc cacccggctg 240
   aagagaaccg ccagaagaag atacaccaga cggaagaacc ggatctgcta tctgcaagag 300
   atcttcagca acgagatggc caaggtggac gacagcttct tccacagact ggaagagtcc 360
   ttcctggtgg aagaggataa gaagcacgag cggcacccca tcttcggcaa catcgtggac 420
   gaggtggcct accacgagaa gtaccccacc atctaccacc tgagaaagaa actggtggac 480
   agcaccgaca aggccgacct gcggctgatc tatctggccc tggcccacat gatcaagttc 540
   cggggccact tcctgatcga gggcgacctg aaccccgaca acagcgacgt ggacaagctg 600
   ttcatccagc tggtgcagac ctacaaccag ctgttcgagg aaaaccccat caacgccagc 660
   ggcgtggacg ccaaggccat cctgtctgcc agactgagca agagcagacg gctggaaaat 720
   ctgatcgccc agctgcccgg cgagaagaag aatggcctgt tcggaaacct gattgccctg 780
   agcctgggcc tgacccccaa cttcaagagc aacttcgacc tggccgagga tgccaaactg 840
   cagctgagca aggacaccta cgacgacgac ctggacaacc tgctggccca gatcggcgac 900
   cagtacgccg acctgtttct ggccgccaag aacctgtccg acgccatcct gctgagcgac 960
   atcctgagag tgaacaccga gatcaccaag gcccccctga gcgcctctat gatcaagaga 1020
   tacgacgagc accaccagga cctgaccctg ctgaaagctc tcgtgcggca gcagctgcct 1080
   gagaagtaca aagagatttt cttcgaccag agcaagaacg gctacgccgg ctacattgac 1140
   ggcggagcca gccaggaaga gttctacaag ttcatcaagc ccatcctgga aaagatggac 1200
   ggcaccgagg aactgctcgt gaagctgaac agagaggacc tgctgcggaa gcagcggacc 1260
   ttcgacaacg gcagcatccc ccaccagatc cacctgggag agctgcacgc cattctgcgg 1320
   cggcaggaag atttttaccc attcctgaag gacaaccggg aaaagatcga gaagatcctg 1380
   accttccgca tcccctacta cgtgggccct ctggccaggg gaaacagcag attcgcctgg 1440
   atgaccagaa agagcgagga aaccatcacc ccctggaact tcgaggaagt ggtggacaag 1500
   ggcgcttccg cccagagctt catcgagcgg atgaccaact tcgataagaa cctgcccaac 1560
   gagaaggtgc tgcccaagca cagcctgctg tacgagtact tcaccgtgta taacgagctg 1620
   accaaagtga aatacgtgac cgagggaatg agaaagcccg ccttcctgag cggcgagcag 1680
   aaaaaggcca tcgtggacct gctgttcaag accaaccgga aagtgaccgt gaagcagctg 1740
   aaagaggact acttcaagaa aatcgagtgc ttcgactccg tggaaatctc cggcgtggaa 1800
   gatcggttca acgcctccct gggcacatac cacgatctgc tgaaaattat caaggacaag 1860
   gacttcctgg acaatgagga aaacgaggac attctggaag atatcgtgct gaccctgaca 1920
   ctgtttgagg acagagagat gatcgaggaa cggctgaaaa cctatgccca cctgttcgac 1980
   gacaaagtga tgaagcagct gaagcggcgg agatacaccg gctggggcag gctgagccgg 2040
   aagctgatca acggcatccg ggacaagcag tccggcaaga caatcctgga tttcctgaag 2100
   tccgacggct tcgccaacag aaacttcatg cagctgatcc acgacgacag cctgaccttt 2160
   aaagaggaca tccagaaagc ccaggtgtcc ggccagggcg atagcctgca cgagcacatt 2220
   gccaatctgg ccggcagccc cgccattaag aagggcatcc tgcagacagt gaaggtggtg 2280
   gacgagctcg tgaaagtgat gggccggcac aagcccgaga acatcgtgat cgaaatggcc 2340
   agagagaacc agaccaccca gaagggacag aagaacagcc gcgagagaat gaagcggatc 2400
   gaagagggca tcaaagagct gggcagccag atcctgaaag aacaccccgt ggaaaacacc 2460
   cagctgcaga acgagaagct gtacctgtac tacctgcaga atgggcggga tatgtacgtg 2520
   gaccaggaac tggacatcaa ccggctgtcc gactacgatg tggaccatat cgtgcctcag 2580
   agctttctga aggacgactc catcgacaac aaggtgctga ccagaagcga caagaaccgg 2640
   ggcaagagcg acaacgtgcc ctccgaagag gtcgtgaaga agatgaagaa ctactggcgg 2700
   cagctgctga acgccaagct gattacccag agaaagttcg acaatctgac caaggccgag 2760
   agaggcggcc tgagcgaact ggataaggcc ggcttcatca agagacagct ggtggaaacc 2820
   cggcagatca caaagcacgt ggcacagatc ctggactccc ggatgaacac taagtacgac 2880
   gagaatgaca agctgatccg ggaagtgaaa gtgatcaccc tgaagtccaa gctggtgtcc 2940
   gatttccgga aggatttcca gttttacaaa gtgcgcgaga tcaacaacta ccaccacgcc 3000
   cacgacgcct acctgaacgc cgtcgtggga accgccctga tcaaaaagta ccctaagctg 3060
   gaaagcgagt tcgtgtacgg cgactacaag gtgtacgacg tgcggaagat gatcgccaag 3120
   agcgagcagg aaatcggcaa ggctaccgcc aagtacttct tctacagcaa catcatgaac 3180
   tttttcaaga ccgagattac cctggccaac ggcgagatcc ggaagcggcc tctgatcgag 3240
   acaaacggcg aaaccgggga gatcgtgtgg gataagggcc gggattttgc caccgtgcgg 3300
   aaagtgctga gcatgcccca agtgaatatc gtgaaaaaga ccgaggtgca gacaggcggc 3360
   ttcagcaaag agtctatcct gcccaagagg aacagcgata agctgatcgc cagaaagaag 3420
   gactgggacc ctaagaagta cggcggcttc gacagcccca ccgtggccta ttctgtgctg 3480
   gtggtggcca aagtggaaaa gggcaagtcc aagaaactga agagtgtgaa agagctgctg 3540
   gggatcacca tcatggaaag aagcagcttc gagaagaatc ccatcgactt tctggaagcc 3600
   aagggctaca aagaagtgaa aaaggacctg atcatcaagc tgcctaagta ctccctgttc 3660
   gagctggaaa acggccggaa gagaatgctg gcctctgccg gcgaactgca gaagggaaac 3720
   gaactggccc tgccctccaa atatgtgaac ttcctgtacc tggccagcca ctatgagaag 3780
   ctgaagggct cccccgagga taatgagcag aaacagctgt ttgtggaaca gcacaagcac 3840
   tacctggacg agatcatcga gcagatcagc gagttctcca agagagtgat cctggccgac 3900
   gctaatctgg acaaagtgct gtccgcctac aacaagcacc gggataagcc catcagagag 3960
   caggccgaga atatcatcca cctgtttacc ctgaccaatc tgggagcccc tgccgccttc 4020
   aagtactttg acaccaccat cgaccggaag aggtacacca gcaccaaaga ggtgctggac 4080
   gccaccctga tccaccagag catcaccggc ctgtacgaga cacggatcga cctgtctcag 4140
   ctgggaggcg acgatccaaa aaagaagaga aaggtaggcg gctctggcgg cggctccgga 4200
   ggctctagcc ccgaggacga gatccagcag ctggaggagg agatcagcca gctggagcag 4260
   aagaacagcc agctgaagga gaagaaccag cagctgaagt actaa 4305
<210> 6
   <211> 4305
   <212> DNA
   <213> Artificial sequence
<220>
   <223> Cas9-P4s
<400> 6
   atggccccaa agaagaagcg gaaggtcggt atccacggag tcccagcagc cgacaagaag      60
   tacagcatcg gcctggacat cggcaccaac tctgtgggct gggccgtgat caccgacgag     120
   tacaaggtgc ccagcaagaa attcaaggtg ctgggcaaca ccgaccggca cagcatcaag     180
   aagaacctga tcggagccct gctgttcgac agcggcgaaa cagccgaggc cacccggctg     240
   aagagaaccg ccagaagaag atacaccaga cggaagaacc ggatctgcta tctgcaagag     300
   atcttcagca acgagatggc caaggtggac gacagcttct tccacagact ggaagagtcc     360
   ttcctggtgg aagaggataa gaagcacgag cggcacccca tcttcggcaa catcgtggac     420
   gaggtggcct accacgagaa gtaccccacc atctaccacc tgagaaagaa actggtggac     480
   agcaccgaca aggccgacct gcggctgatc tatctggccc tggcccacat gatcaagttc     540
   cggggccact tcctgatcga gggcgacctg aaccccgaca acagcgacgt ggacaagctg     600
   ttcatccagc tggtgcagac ctacaaccag ctgttcgagg aaaaccccat caacgccagc     660
   ggcgtggacg ccaaggccat cctgtctgcc agactgagca agagcagacg gctggaaaat     720
   ctgatcgccc agctgcccgg cgagaagaag aatggcctgt tcggaaacct gattgccctg     780
   agcctgggcc tgacccccaa cttcaagagc aacttcgacc tggccgagga tgccaaactg     840
   cagctgagca aggacaccta cgacgacgac ctggacaacc tgctggccca gatcggcgac     900
   cagtacgccg acctgtttct ggccgccaag aacctgtccg acgccatcct gctgagcgac     960
   atcctgagag tgaacaccga gatcaccaag gcccccctga gcgcctctat gatcaagaga    1020
   tacgacgagc accaccagga cctgaccctg ctgaaagctc tcgtgcggca gcagctgcct    1080
   gagaagtaca aagagatttt cttcgaccag agcaagaacg gctacgccgg ctacattgac    1140
   ggcggagcca gccaggaaga gttctacaag ttcatcaagc ccatcctgga aaagatggac    1200
   ggcaccgagg aactgctcgt gaagctgaac agagaggacc tgctgcggaa gcagcggacc    1260
   ttcgacaacg gcagcatccc ccaccagatc cacctgggag agctgcacgc cattctgcgg    1320
   cggcaggaag atttttaccc attcctgaag gacaaccggg aaaagatcga gaagatcctg    1380
   accttccgca tcccctacta cgtgggccct ctggccaggg gaaacagcag attcgcctgg 1440
   atgaccagaa agagcgagga aaccatcacc ccctggaact tcgaggaagt ggtggacaag 1500
   ggcgcttccg cccagagctt catcgagcgg atgaccaact tcgataagaa cctgcccaac 1560
   gagaaggtgc tgcccaagca cagcctgctg tacgagtact tcaccgtgta taacgagctg 1620
   accaaagtga aatacgtgac cgagggaatg agaaagcccg ccttcctgag cggcgagcag 1680
   aaaaaggcca tcgtggacct gctgttcaag accaaccgga aagtgaccgt gaagcagctg 1740
   aaagaggact acttcaagaa aatcgagtgc ttcgactccg tggaaatctc cggcgtggaa 1800
   gatcggttca acgcctccct gggcacatac cacgatctgc tgaaaattat caaggacaag 1860
   gacttcctgg acaatgagga aaacgaggac attctggaag atatcgtgct gaccctgaca 1920
   ctgtttgagg acagagagat gatcgaggaa cggctgaaaa cctatgccca cctgttcgac 1980
   gacaaagtga tgaagcagct gaagcggcgg agatacaccg gctggggcag gctgagccgg 2040
   aagctgatca acggcatccg ggacaagcag tccggcaaga caatcctgga tttcctgaag 2100
   tccgacggct tcgccaacag aaacttcatg cagctgatcc acgacgacag cctgaccttt 2160
   aaagaggaca tccagaaagc ccaggtgtcc ggccagggcg atagcctgca cgagcacatt 2220
   gccaatctgg ccggcagccc cgccattaag aagggcatcc tgcagacagt gaaggtggtg 2280
   gacgagctcg tgaaagtgat gggccggcac aagcccgaga acatcgtgat cgaaatggcc 2340
   agagagaacc agaccaccca gaagggacag aagaacagcc gcgagagaat gaagcggatc 2400
   gaagagggca tcaaagagct gggcagccag atcctgaaag aacaccccgt ggaaaacacc 2460
   cagctgcaga acgagaagct gtacctgtac tacctgcaga atgggcggga tatgtacgtg 2520
   gaccaggaac tggacatcaa ccggctgtcc gactacgatg tggaccatat cgtgcctcag 2580
   agctttctga aggacgactc catcgacaac aaggtgctga ccagaagcga caagaaccgg 2640
   ggcaagagcg acaacgtgcc ctccgaagag gtcgtgaaga agatgaagaa ctactggcgg 2700
   cagctgctga acgccaagct gattacccag agaaagttcg acaatctgac caaggccgag 2760
   agaggcggcc tgagcgaact ggataaggcc ggcttcatca agagacagct ggtggaaacc 2820
   cggcagatca caaagcacgt ggcacagatc ctggactccc ggatgaacac taagtacgac 2880
   gagaatgaca agctgatccg ggaagtgaaa gtgatcaccc tgaagtccaa gctggtgtcc 2940
   gatttccgga aggatttcca gttttacaaa gtgcgcgaga tcaacaacta ccaccacgcc 3000
   cacgacgcct acctgaacgc cgtcgtggga accgccctga tcaaaaagta ccctaagctg 3060
   gaaagcgagt tcgtgtacgg cgactacaag gtgtacgacg tgcggaagat gatcgccaag 3120
   agcgagcagg aaatcggcaa ggctaccgcc aagtacttct tctacagcaa catcatgaac 3180
   tttttcaaga ccgagattac cctggccaac ggcgagatcc ggaagcggcc tctgatcgag 3240
   acaaacggcg aaaccgggga gatcgtgtgg gataagggcc gggattttgc caccgtgcgg 3300
   aaagtgctga gcatgcccca agtgaatatc gtgaaaaaga ccgaggtgca gacaggcggc 3360
   ttcagcaaag agtctatcct gcccaagagg aacagcgata agctgatcgc cagaaagaag 3420
   gactgggacc ctaagaagta cggcggcttc gacagcccca ccgtggccta ttctgtgctg 3480
   gtggtggcca aagtggaaaa gggcaagtcc aagaaactga agagtgtgaa agagctgctg 3540
   gggatcacca tcatggaaag aagcagcttc gagaagaatc ccatcgactt tctggaagcc 3600
   aagggctaca aagaagtgaa aaaggacctg atcatcaagc tgcctaagta ctccctgttc 3660
   gagctggaaa acggccggaa gagaatgctg gcctctgccg gcgaactgca gaagggaaac 3720
   gaactggccc tgccctccaa atatgtgaac ttcctgtacc tggccagcca ctatgagaag 3780
   ctgaagggct cccccgagga taatgagcag aaacagctgt ttgtggaaca gcacaagcac 3840
   tacctggacg agatcatcga gcagatcagc gagttctcca agagagtgat cctggccgac 3900
   gctaatctgg acaaagtgct gtccgcctac aacaagcacc gggataagcc catcagagag 3960
   caggccgaga atatcatcca cctgtttacc ctgaccaatc tgggagcccc tgccgccttc 4020
   aagtactttg acaccaccat cgaccggaag aggtacacca gcaccaaaga ggtgctggac 4080
   gccaccctga tccaccagag catcaccggc ctgtacgaga cacggatcga cctgtctcag 4140
   ctgggaggcg acgatccaaa aaagaagaga aaggtaggcg gctctggcgg cggctccgga 4200
   ggctctagcc ccgaggacaa gatcagccag ctgaagcaga agatccagca gctgaagcag 4260
   gagaaccagc agctggagga ggagaacagc cagctggagt actaa 4305
<210> 7
   <211> 4305
   <212> DNA
   <213> Artificial sequence
<220>
   <223> Cas9-AP4
<400> 7
   atggccccaa agaagaagcg gaaggtcggt atccacggag tcccagcagc cgacaagaag 60
   tacagcatcg gcctggacat cggcaccaac tctgtgggct gggccgtgat caccgacgag 120
   tacaaggtgc ccagcaagaa attcaaggtg ctgggcaaca ccgaccggca cagcatcaag    180
   aagaacctga tcggagccct gctgttcgac agcggcgaaa cagccgaggc cacccggctg    240
   aagagaaccg ccagaagaag atacaccaga cggaagaacc ggatctgcta tctgcaagag    300
   atcttcagca acgagatggc caaggtggac gacagcttct tccacagact ggaagagtcc    360
   ttcctggtgg aagaggataa gaagcacgag cggcacccca tcttcggcaa catcgtggac    420
   gaggtggcct accacgagaa gtaccccacc atctaccacc tgagaaagaa actggtggac    480
   agcaccgaca aggccgacct gcggctgatc tatctggccc tggcccacat gatcaagttc    540
   cggggccact tcctgatcga gggcgacctg aaccccgaca acagcgacgt ggacaagctg    600
   ttcatccagc tggtgcagac ctacaaccag ctgttcgagg aaaaccccat caacgccagc    660
   ggcgtggacg ccaaggccat cctgtctgcc agactgagca agagcagacg gctggaaaat    720
   ctgatcgccc agctgcccgg cgagaagaag aatggcctgt tcggaaacct gattgccctg    780
   agcctgggcc tgacccccaa cttcaagagc aacttcgacc tggccgagga tgccaaactg    840
   cagctgagca aggacaccta cgacgacgac ctggacaacc tgctggccca gatcggcgac    900
   cagtacgccg acctgtttct ggccgccaag aacctgtccg acgccatcct gctgagcgac    960
   atcctgagag tgaacaccga gatcaccaag gcccccctga gcgcctctat gatcaagaga    1020
   tacgacgagc accaccagga cctgaccctg ctgaaagctc tcgtgcggca gcagctgcct    1080
   gagaagtaca aagagatttt cttcgaccag agcaagaacg gctacgccgg ctacattgac    1140
   ggcggagcca gccaggaaga gttctacaag ttcatcaagc ccatcctgga aaagatggac    1200
   ggcaccgagg aactgctcgt gaagctgaac agagaggacc tgctgcggaa gcagcggacc    1260
   ttcgacaacg gcagcatccc ccaccagatc cacctgggag agctgcacgc cattctgcgg    1320
   cggcaggaag atttttaccc attcctgaag gacaaccggg aaaagatcga gaagatcctg    1380
   accttccgca tcccctacta cgtgggccct ctggccaggg gaaacagcag attcgcctgg    1440
   atgaccagaa agagcgagga aaccatcacc ccctggaact tcgaggaagt ggtggacaag    1500
   ggcgcttccg cccagagctt catcgagcgg atgaccaact tcgataagaa cctgcccaac    1560
   gagaaggtgc tgcccaagca cagcctgctg tacgagtact tcaccgtgta taacgagctg    1620
   accaaagtga aatacgtgac cgagggaatg agaaagcccg ccttcctgag cggcgagcag    1680
   aaaaaggcca tcgtggacct gctgttcaag accaaccgga aagtgaccgt gaagcagctg    1740
   aaagaggact acttcaagaa aatcgagtgc ttcgactccg tggaaatctc cggcgtggaa    1800
   gatcggttca acgcctccct gggcacatac cacgatctgc tgaaaattat caaggacaag 1860
   gacttcctgg acaatgagga aaacgaggac attctggaag atatcgtgct gaccctgaca 1920
   ctgtttgagg acagagagat gatcgaggaa cggctgaaaa cctatgccca cctgttcgac 1980
   gacaaagtga tgaagcagct gaagcggcgg agatacaccg gctggggcag gctgagccgg 2040
   aagctgatca acggcatccg ggacaagcag tccggcaaga caatcctgga tttcctgaag 2100
   tccgacggct tcgccaacag aaacttcatg cagctgatcc acgacgacag cctgaccttt 2160
   aaagaggaca tccagaaagc ccaggtgtcc ggccagggcg atagcctgca cgagcacatt 2220
   gccaatctgg ccggcagccc cgccattaag aagggcatcc tgcagacagt gaaggtggtg 2280
   gacgagctcg tgaaagtgat gggccggcac aagcccgaga acatcgtgat cgaaatggcc 2340
   agagagaacc agaccaccca gaagggacag aagaacagcc gcgagagaat gaagcggatc 2400
   gaagagggca tcaaagagct gggcagccag atcctgaaag aacaccccgt ggaaaacacc 2460
   cagctgcaga acgagaagct gtacctgtac tacctgcaga atgggcggga tatgtacgtg 2520
   gaccaggaac tggacatcaa ccggctgtcc gactacgatg tggaccatat cgtgcctcag 2580
   agctttctga aggacgactc catcgacaac aaggtgctga ccagaagcga caagaaccgg 2640
   ggcaagagcg acaacgtgcc ctccgaagag gtcgtgaaga agatgaagaa ctactggcgg 2700
   cagctgctga acgccaagct gattacccag agaaagttcg acaatctgac caaggccgag 2760
   agaggcggcc tgagcgaact ggataaggcc ggcttcatca agagacagct ggtggaaacc 2820
   cggcagatca caaagcacgt ggcacagatc ctggactccc ggatgaacac taagtacgac 2880
   gagaatgaca agctgatccg ggaagtgaaa gtgatcaccc tgaagtccaa gctggtgtcc 2940
   gatttccgga aggatttcca gttttacaaa gtgcgcgaga tcaacaacta ccaccacgcc 3000
   cacgacgcct acctgaacgc cgtcgtggga accgccctga tcaaaaagta ccctaagctg 3060
   gaaagcgagt tcgtgtacgg cgactacaag gtgtacgacg tgcggaagat gatcgccaag 3120
   agcgagcagg aaatcggcaa ggctaccgcc aagtacttct tctacagcaa catcatgaac 3180
   tttttcaaga ccgagattac cctggccaac ggcgagatcc ggaagcggcc tctgatcgag 3240
   acaaacggcg aaaccgggga gatcgtgtgg gataagggcc gggattttgc caccgtgcgg 3300
   aaagtgctga gcatgcccca agtgaatatc gtgaaaaaga ccgaggtgca gacaggcggc 3360
   ttcagcaaag agtctatcct gcccaagagg aacagcgata agctgatcgc cagaaagaag 3420
   gactgggacc ctaagaagta cggcggcttc gacagcccca ccgtggccta ttctgtgctg 3480
   gtggtggcca aagtggaaaa gggcaagtcc aagaaactga agagtgtgaa agagctgctg 3540
   gggatcacca tcatggaaag aagcagcttc gagaagaatc ccatcgactt tctggaagcc 3600
   aagggctaca aagaagtgaa aaaggacctg atcatcaagc tgcctaagta ctccctgttc 3660
   gagctggaaa acggccggaa gagaatgctg gcctctgccg gcgaactgca gaagggaaac 3720
   gaactggccc tgccctccaa atatgtgaac ttcctgtacc tggccagcca ctatgagaag 3780
   ctgaagggct cccccgagga taatgagcag aaacagctgt ttgtggaaca gcacaagcac 3840
   tacctggacg agatcatcga gcagatcagc gagttctcca agagagtgat cctggccgac 3900
   gctaatctgg acaaagtgct gtccgcctac aacaagcacc gggataagcc catcagagag 3960
   caggccgaga atatcatcca cctgtttacc ctgaccaatc tgggagcccc tgccgccttc 4020
   aagtactttg acaccaccat cgaccggaag aggtacacca gcaccaaaga ggtgctggac 4080
   gccaccctga tccaccagag catcaccggc ctgtacgaga cacggatcga cctgtctcag 4140
   ctgggaggcg acgatccaaa aaagaagaga aaggtaggcg gctctggcgg cggctccgga 4200
   ggctctagcc ccgaggacga gctggccgcc aacgaggagg agctgcagca gaacgagcag 4260
   aagctggccc agatcaagca gaagctgcag gccatcaagt actaa 4305
<210> 8
   <211> 957
   <212> DNA
   <213> Artificial sequence
<220>
   <223> P4-EXOIII
<400> 8
   agcccggaag ataaaattgc tcagctgaaa caaaaaatcc aagcgctgaa acaggaaaac 60
   cagcagctgg aagaggaaaa cgccgcactg gaatatggtg gcggctctgg cggcggctcc 120
   ggaggctctg atccaaaaaa gaagagaaag gtaaaatttg tctcttttaa tatcaacggc 180
   ctgcgcgcca gacctcacca gcttgaagcc atcgtcgaaa agcaccaacc ggatgtgatt 240
   ggcctgcagg agacaaaagt tcatgacgat atgtttccgc tcgaagaggt ggcgaagctc 300
   ggctacaacg tgttttatca cgggcagaaa ggccattatg gcgtggcgct gctgaccaaa 360
   gagacgccga ttgccgtgcg tcgcggcttt cccggtgacg acgaagaggc gcagcggcgg 420
   attattatgg cggaaatccc ctcactgctg ggtaatgtca ccgtgatcaa cggttacttc 480
   ccgcagggtg aaagccgcga ccatccgata aaattcccgg caaaagcgca gttttatcag 540
   aatctgcaaa actacctgga aaccgaactc aaacgtgata atccggtact gattatgggc 600
   gatatgaata tcagccctac agatctggac atcggcattg gcgaagaaaa ccgtaagcgc 660
   tggctgcgta ccggtaaatg ctctttcctg ccggaagagc gcgaatggat ggacaggctg 720
   atgagctggg ggttggtcga taccttccgc catgcgaatc cgcaaacagc agatcgtttc 780
   tcatggtttg attaccgctc aaaaggtttt gacgataacc gtggtctgcg catcgacctg 840
   ctgctcgcca gccaaccgct ggcagaatgt tgcgtagaaa ccggcatcga ctatgaaatc 900
   cgcagcatgg aaaaaccgtc cgatcacgcc cccgtctggg cgaccttccg ccgctaa 957
<210> 9
   <211> 957
   <212> DNA
   <213> Artificial sequence
<220>
   <223> P3-EXOIII
<400> 9
   tccccggaag atgagatcca gcaactggaa gaagaaatcg ctcagctgga acagaaaaac 60
   gcagcgctga aagagaaaaa ccaggcgctg aaatacggtg gcggctctgg cggcggctcc 120
   ggaggctctg atccaaaaaa gaagagaaag gtaaaatttg tctcttttaa tatcaacggc 180
   ctgcgcgcca gacctcacca gcttgaagcc atcgtcgaaa agcaccaacc ggatgtgatt 240
   ggcctgcagg agacaaaagt tcatgacgat atgtttccgc tcgaagaggt ggcgaagctc 300
   ggctacaacg tgttttatca cgggcagaaa ggccattatg gcgtggcgct gctgaccaaa 360
   gagacgccga ttgccgtgcg tcgcggcttt cccggtgacg acgaagaggc gcagcggcgg 420
   attattatgg cggaaatccc ctcactgctg ggtaatgtca ccgtgatcaa cggttacttc 480
   ccgcagggtg aaagccgcga ccatccgata aaattcccgg caaaagcgca gttttatcag 540
   aatctgcaaa actacctgga aaccgaactc aaacgtgata atccggtact gattatgggc 600
   gatatgaata tcagccctac agatctggac atcggcattg gcgaagaaaa ccgtaagcgc 660
   tggctgcgta ccggtaaatg ctctttcctg ccggaagagc gcgaatggat ggacaggctg 720
   atgagctggg ggttggtcga taccttccgc catgcgaatc cgcaaacagc agatcgtttc 780
   tcatggtttg attaccgctc aaaaggtttt gacgataacc gtggtctgcg catcgacctg 840
   ctgctcgcca gccaaccgct ggcagaatgt tgcgtagaaa ccggcatcga ctatgaaatc 900
   cgcagcatgg aaaaaccgtc cgatcacgcc cccgtctggg cgaccttccg ccgctaa 957
<210> 10
   <211> 939
   <212> DNA
   <213> Artificial sequence
<220>
   <223> N5-EXOIII
<400> 10
   gagatcgccg ccctggaggc caagatcgcc gccctgaagg ccaagaacgc cgccctgaag      60
   gccgagatcg ccgccctgga ggccggcggc tctggcggcg gctccggagg ctctgatcca     120
   aaaaagaaga gaaaggtaaa atttgtctct tttaatatca acggcctgcg cgccagacct     180
   caccagcttg aagccatcgt cgaaaagcac caaccggatg tgattggcct gcaggagaca     240
   aaagttcatg acgatatgtt tccgctcgaa gaggtggcga agctcggcta caacgtgttt     300
   tatcacgggc agaaaggcca ttatggcgtg gcgctgctga ccaaagagac gccgattgcc     360
   gtgcgtcgcg gctttcccgg tgacgacgaa gaggcgcagc ggcggattat tatggcggaa     420
   atcccctcac tgctgggtaa tgtcaccgtg atcaacggtt acttcccgca gggtgaaagc     480
   cgcgaccatc cgataaaatt cccggcaaaa gcgcagtttt atcagaatct gcaaaactac     540
   ctggaaaccg aactcaaacg tgataatccg gtactgatta tgggcgatat gaatatcagc     600
   cctacagatc tggacatcgg cattggcgaa gaaaaccgta agcgctggct gcgtaccggt     660
   aaatgctctt tcctgccgga agagcgcgaa tggatggaca ggctgatgag ctgggggttg     720
   gtcgatacct tccgccatgc gaatccgcaa acagcagatc gtttctcatg gtttgattac     780
   cgctcaaaag gttttgacga taaccgtggt ctgcgcatcg acctgctgct cgccagccaa     840
   ccgctggcag aatgttgcgt agaaaccggc atcgactatg aaatccgcag catggaaaaa     900
   ccgtccgatc acgcccccgt ctgggcgacc ttccgccgc                            939
<210> 11
   <211> 939
   <212> DNA
   <213> Artificial sequence
<220>
   <223> N6-EXOIII
<400> 11
   aagatcgccg ccctgaaggc cgagatcgcc gccctggagg ccgagaacgc cgccctggag      60
   gccaagatcg ccgccctgaa ggccggcggc tctggcggcg gctccggagg ctctgatcca     120
   aaaaagaaga gaaaggtaaa atttgtctct tttaatatca acggcctgcg cgccagacct 180
   caccagcttg aagccatcgt cgaaaagcac caaccggatg tgattggcct gcaggagaca 240
   aaagttcatg acgatatgtt tccgctcgaa gaggtggcga agctcggcta caacgtgttt 300
   tatcacgggc agaaaggcca ttatggcgtg gcgctgctga ccaaagagac gccgattgcc 360
   gtgcgtcgcg gctttcccgg tgacgacgaa gaggcgcagc ggcggattat tatggcggaa 420
   atcccctcac tgctgggtaa tgtcaccgtg atcaacggtt acttcccgca gggtgaaagc 480
   cgcgaccatc cgataaaatt cccggcaaaa gcgcagtttt atcagaatct gcaaaactac 540
   ctggaaaccg aactcaaacg tgataatccg gtactgatta tgggcgatat gaatatcagc 600
   cctacagatc tggacatcgg cattggcgaa gaaaaccgta agcgctggct gcgtaccggt 660
   aaatgctctt tcctgccgga agagcgcgaa tggatggaca ggctgatgag ctgggggttg 720
   gtcgatacct tccgccatgc gaatccgcaa acagcagatc gtttctcatg gtttgattac 780
   cgctcaaaag gttttgacga taaccgtggt ctgcgcatcg acctgctgct cgccagccaa 840
   ccgctggcag aatgttgcgt agaaaccggc atcgactatg aaatccgcag catggaaaaa 900
   ccgtccgatc acgcccccgt ctgggcgacc ttccgccgc 939
<210> 12
   <211> 951
   <212> DNA
   <213> Artificial sequence
<220>
   <223> P3S-EXOIII
<400> 12
   agccccgagg acgagatcca gcagctggag gaggagatca gccagctgga gcagaagaac 60
   agccagctga aggagaagaa ccagcagctg aagtacggcg gctctggcgg cggctccgga 120
   ggctctgatc caaaaaagaa gagaaaggta aaatttgtct cttttaatat caacggcctg 180
   cgcgccagac ctcaccagct tgaagccatc gtcgaaaagc accaaccgga tgtgattggc 240
   ctgcaggaga caaaagttca tgacgatatg tttccgctcg aagaggtggc gaagctcggc 300
   tacaacgtgt tttatcacgg gcagaaaggc cattatggcg tggcgctgct gaccaaagag 360
   acgccgattg ccgtgcgtcg cggctttccc ggtgacgacg aagaggcgca gcggcggatt 420
   attatggcgg aaatcccctc actgctgggt aatgtcaccg tgatcaacgg ttacttcccg 480
   cagggtgaaa gccgcgacca tccgataaaa ttcccggcaa aagcgcagtt ttatcagaat 540
   ctgcaaaact acctggaaac cgaactcaaa cgtgataatc cggtactgat tatgggcgat 600
   atgaatatca gccctacaga tctggacatc ggcattggcg aagaaaaccg taagcgctgg 660
   ctgcgtaccg gtaaatgctc tttcctgccg gaagagcgcg aatggatgga caggctgatg 720
   agctgggggt tggtcgatac cttccgccat gcgaatccgc aaacagcaga tcgtttctca 780
   tggtttgatt accgctcaaa aggttttgac gataaccgtg gtctgcgcat cgacctgctg 840
   ctcgccagcc aaccgctggc agaatgttgc gtagaaaccg gcatcgacta tgaaatccgc 900
   agcatggaaa aaccgtccga tcacgccccc gtctgggcga ccttccgccg c 951
<210> 13
   <211> 951
   <212> DNA
   <213> Artificial sequence
<220>
   <223> P4S-EXOIII
<400> 13
   agccccgagg acaagatcag ccagctgaag cagaagatcc agcagctgaa gcaggagaac 60
   cagcagctgg aggaggagaa cagccagctg gagtacggcg gctctggcgg cggctccgga 120
   ggctctgatc caaaaaagaa gagaaaggta aaatttgtct cttttaatat caacggcctg 180
   cgcgccagac ctcaccagct tgaagccatc gtcgaaaagc accaaccgga tgtgattggc 240
   ctgcaggaga caaaagttca tgacgatatg tttccgctcg aagaggtggc gaagctcggc 300
   tacaacgtgt tttatcacgg gcagaaaggc cattatggcg tggcgctgct gaccaaagag 360
   acgccgattg ccgtgcgtcg cggctttccc ggtgacgacg aagaggcgca gcggcggatt 420
   attatggcgg aaatcccctc actgctgggt aatgtcaccg tgatcaacgg ttacttcccg 480
   cagggtgaaa gccgcgacca tccgataaaa ttcccggcaa aagcgcagtt ttatcagaat 540
   ctgcaaaact acctggaaac cgaactcaaa cgtgataatc cggtactgat tatgggcgat 600
   atgaatatca gccctacaga tctggacatc ggcattggcg aagaaaaccg taagcgctgg 660
   ctgcgtaccg gtaaatgctc tttcctgccg gaagagcgcg aatggatgga caggctgatg 720
   agctgggggt tggtcgatac cttccgccat gcgaatccgc aaacagcaga tcgtttctca 780
   tggtttgatt accgctcaaa aggttttgac gataaccgtg gtctgcgcat cgacctgctg 840
   ctcgccagcc aaccgctggc agaatgttgc gtagaaaccg gcatcgacta tgaaatccgc 900
   agcatggaaa aaccgtccga tcacgccccc gtctgggcga ccttccgccg c 951
<210> 14
   <211> 951
   <212> DNA
   <213> Artificial sequence
<220>
   <223> AP4-EXOIII
<400> 14
   agccccgagg acgagctggc cgccaacgag gaggagctgc agcagaacga gcagaagctg      60
   gcccagatca agcagaagct gcaggccatc aagtacggcg gctctggcgg cggctccgga     120
   ggctctgatc caaaaaagaa gagaaaggta aaatttgtct cttttaatat caacggcctg     180
   cgcgccagac ctcaccagct tgaagccatc gtcgaaaagc accaaccgga tgtgattggc     240
   ctgcaggaga caaaagttca tgacgatatg tttccgctcg aagaggtggc gaagctcggc     300
   tacaacgtgt tttatcacgg gcagaaaggc cattatggcg tggcgctgct gaccaaagag     360
   acgccgattg ccgtgcgtcg cggctttccc ggtgacgacg aagaggcgca gcggcggatt     420
   attatggcgg aaatcccctc actgctgggt aatgtcaccg tgatcaacgg ttacttcccg     480
   cagggtgaaa gccgcgacca tccgataaaa ttcccggcaa aagcgcagtt ttatcagaat     540
   ctgcaaaact acctggaaac cgaactcaaa cgtgataatc cggtactgat tatgggcgat     600
   atgaatatca gccctacaga tctggacatc ggcattggcg aagaaaaccg taagcgctgg     660
   ctgcgtaccg gtaaatgctc tttcctgccg gaagagcgcg aatggatgga caggctgatg     720
   agctgggggt tggtcgatac cttccgccat gcgaatccgc aaacagcaga tcgtttctca     780
   tggtttgatt accgctcaaa aggttttgac gataaccgtg gtctgcgcat cgacctgctg     840
   ctcgccagcc aaccgctggc agaatgttgc gtagaaaccg gcatcgacta tgaaatccgc     900
   agcatggaaa aaccgtccga tcacgccccc gtctgggcga ccttccgccg c              951
<210> 15
   <211> 840
   <212> DNA
   <213> Artificial sequence
<220>
   <223> P3-TREX2
<400> 15
   tccccggaag atgagatcca gcaactggaa gaagaaatcg ctcagctgga acagaaaaac      60
   gcagcgctga aagagaaaaa ccaggcgctg aaatacggtg gcggctctgg cggcggctcc     120
   ggaggctcta tgtctgagcc acctcgggct gagacctttg tattcctgga cctagaagcc      180
   actgggctcc caaacatgga ccctgagatt gcagagatat ccctttttgc tgttcaccgc      240
   tcttccctgg agaacccaga acgggatgat tctggttcct tggtgctgcc ccgtgttctg      300
   gacaagctca cactgtgcat gtgcccggag cgccccttta ctgccaaggc cagtgagatt      360
   actggtttga gcagcgaaag cctgatgcac tgcgggaagg ctggtttcaa tggcgctgtg      420
   gtaaggacac tgcagggctt cctaagccgc caggagggcc ccatctgcct tgtggcccac      480
   aatggcttcg attatgactt cccactgctg tgcacggagc tacaacgtct gggtgcccat      540
   ctgccccaag acactgtctg cctggacaca ctgcctgcat tgcggggcct ggaccgtgct      600
   cacagccacg gcaccagggc tcaaggccgc aaaagctaca gcctggccag tctcttccac      660
   cgctacttcc aggctgaacc cagtgctgcc cattcagcag aaggtgatgt gcacaccctg      720
   cttctgatct tcctgcatcg tgctcctgag ctgctcgcct gggcagatga gcaggcccgc      780
   agctgggctc atattgagcc catgtacgtg ccacctgatg gtccaagcct cgaagcctga      840
<210> 16
   <211> 837
   <212> DNA
   <213> Artificial sequence
<220>
   <223> P4-TREX2
<400> 16
   agcccggaag ataaaattgc tcagctgaaa caaaaaatcc aagcgctgaa acaggaaaac       60
   cagcagctgg aagaggaaaa cgccgcactg gaatatggtg gcggctctgg cggcggctcc      120
   ggaggctctt ctgagccacc tcgggctgag acctttgtat tcctggacct agaagccact      180
   gggctcccaa acatggaccc tgagattgca gagatatccc tttttgctgt tcaccgctct      240
   tccctggaga acccagaacg ggatgattct ggttccttgg tgctgccccg tgttctggac      300
   aagctcacac tgtgcatgtg cccggagcgc ccctttactg ccaaggccag tgagattact      360
   ggtttgagca gcgaaagcct gatgcactgc gggaaggctg gtttcaatgg cgctgtggta      420
   aggacactgc agggcttcct aagccgccag gagggcccca tctgccttgt ggcccacaat      480
   ggcttcgatt atgacttccc actgctgtgc acggagctac aacgtctggg tgcccatctg      540
   ccccaagaca ctgtctgcct ggacacactg cctgcattgc ggggcctgga ccgtgctcac      600
   agccacggca ccagggctca aggccgcaaa agctacagcc tggccagtct cttccaccgc      660
   tacttccagg ctgaacccag tgctgcccat tcagcagaag gtgatgtgca caccctgctt 720
   ctgatcttcc tgcatcgtgc tcctgagctg ctcgcctggg cagatgagca ggcccgcagc 780
   tgggctcata ttgagcccat gtacgtgcca cctgatggtc caagcctcga agcctga 837
<210> 17
   <211> 2668
   <212> DNA
   <213> Artificial sequence
<220>
   <223> P3-EXO1
<400> 17
   tccccggaag atgagatcca gcaactggaa gaagaaatcg ctcagctgga acagaaaaac 60
   gcagcgctga aagagaaaaa ccaggcgctg aaatacggtg gcggctctgg cggcggctcc 120
   ggaggctctg cgatcgccat ggggatacag ggattgctac aatttatcaa agaagcttca 180
   gaacccatcc atgtgaggaa gtataaaggg caggtagtag ctgtggatac atattgctgg 240
   cttcacaaag gagctattgc ttgtgctgaa aaactagcca aaggtgaacc tactgatagg 300
   tatgtaggat tttgtatgaa atttgtaaat atgttactat ctcatgggat caagcctatt 360
   ctcgtatttg atggatgtac tttaccttct aaaaaggaag tagagagatc tagaagagaa 420
   agacgacaag ccaatcttct taagggaaag caacttcttc gtgaggggaa agtctcggaa 480
   gctcgagagt gtttcacccg gtctatcaat atcacacatg ccatggccca caaagtaatt 540
   aaagctgccc ggtctcaggg ggtagattgc ctcgtggctc cctatgaagc tgatgcgcag 600
   ttggcctatc ttaacaaagc gggaattgtg caagccataa ttacagagga ctcggatctc 660
   ctagcttttg gctgtaaaaa ggtaatttta aagatggacc agtttggaaa tggacttgaa 720
   attgatcaag ctcggctagg aatgtgcaga cagcttgggg atgtattcac ggaagagaag 780
   tttcgttaca tgtgtattct ttcaggttgt gactacctgt catcactgcg tgggattgga 840
   ttagcaaagg catgcaaagt cctaagacta gccaataatc cagatatagt aaaggttatc 900
   aagaaaattg gacattatct caagatgaat atcacggtac cagaggatta catcaacggg 960
   tttattcggg ccaacaatac cttcctctat cagctagttt ttgatcccat caaaaggaaa 1020
   cttattcctc tgaacgccta tgaagatgat gttgatcctg aaacactaag ctacgctggg 1080
   caatatgttg atgattccat agctcttcaa atagcacttg gaaataaaga tataaatact 1140
   tttgaacaga tcgatgacta caatccagac actgctatgc ctgcccattc aagaagtcat 1200
   agttgggatg acaaaacatg tcaaaagtca gctaatgtta gcagcatttg gcataggaat 1260
   tactctccca gaccagagtc gggtactgtt tcagatgccc cacaattgaa ggaaaatcca 1320
   agtactgtgg gagtggaacg agtgattagt actaaagggt taaatctccc aaggaaatca 1380
   tccattgtga aaagaccaag aagtgcagag ctgtcagaag atgacctgtt gagtcagtat 1440
   tctctttcat ttacgaagaa gaccaagaaa aatagctctg aaggcaataa atcattgagc 1500
   ttttctgaag tgtttgtgcc tgacctggta aatggaccta ctaacaaaaa gagtgtaagc 1560
   actccaccta ggacgagaaa taaatttgca acatttttac aaaggaaaaa tgaagaaagt 1620
   ggtgcagttg tggttccagg gaccagaagc aggttttttt gcagttcaga ttctactgac 1680
   tgtgtatcaa acaaagtgag catccagcct ctggatgaaa ctgctgtcac agataaagag 1740
   aacaatctgc atgaatcaga gtatggagac caagaaggca agagactggt tgacacagat 1800
   gtagcacgta attcaagtga tgacattccg aataatcata ttccaggtga tcatattcca 1860
   gacaaggcaa cagtgtttac agatgaagag tcctactctt ttgagagcag caaatttaca 1920
   aggaccattt caccacccac tttgggaaca ctaagaagtt gttttagttg gtctggaggt 1980
   cttggagatt tttcaagaac gccgagcccc tctccaagca cagcattgca gcagttccga 2040
   agaaagagcg attcccccac ctctttgcct gagaataata tgtctgatgt gtcgcagtta 2100
   aagagcgagg agtccagtga cgatgagtct catcccttac gagaaggggc atgttcttca 2160
   cagtcccagg aaagtggaga attctcactg cagagttcaa atgcatcaaa gctttctcag 2220
   tgctctagta aggactctga ttcagaggaa tctgattgca atattaagtt acttgacagt 2280
   caaagtgacc agacctccaa gctatgttta tctcatttct caaaaaaaga cacacctcta 2340
   aggaacaagg ttcctgggct atataagtcc agttctgcag actctctttc tacaaccaag 2400
   atcaaacctc taggacctgc cagagccagt gggctgagca agaagccggc aagcatccag 2460
   aagagaaagc atcataatgc cgagaacaag ccggggttac agatcaaact caatgagctc 2520
   tggaaaaact ttggatttaa aaaagattct gaaaagcttc ctccttgtaa gaaacccctg 2580
   tccccagtca gagataacat ccaactaact ccagaagcgg aagaggatat atttaacaaa 2640
   cctgaatgtg gccgtgttca aagagcaa 2668
<210> 18
   <211> 2668
   <212> DNA
   <213> Artificial sequence
<220>
   <223> P4-EXO1
<400> 18
   agcccggaag ataaaattgc tcagctgaaa caaaaaatcc aagcgctgaa acaggaaaac      60
   cagcagctgg aagaggaaaa cgccgcactg gaatatggtg gcggctctgg cggcggctcc     120
   ggaggctctg cgatcgccat ggggatacag ggattgctac aatttatcaa agaagcttca     180
   gaacccatcc atgtgaggaa gtataaaggg caggtagtag ctgtggatac atattgctgg     240
   cttcacaaag gagctattgc ttgtgctgaa aaactagcca aaggtgaacc tactgatagg     300
   tatgtaggat tttgtatgaa atttgtaaat atgttactat ctcatgggat caagcctatt     360
   ctcgtatttg atggatgtac tttaccttct aaaaaggaag tagagagatc tagaagagaa     420
   agacgacaag ccaatcttct taagggaaag caacttcttc gtgaggggaa agtctcggaa     480
   gctcgagagt gtttcacccg gtctatcaat atcacacatg ccatggccca caaagtaatt     540
   aaagctgccc ggtctcaggg ggtagattgc ctcgtggctc cctatgaagc tgatgcgcag     600
   ttggcctatc ttaacaaagc gggaattgtg caagccataa ttacagagga ctcggatctc     660
   ctagcttttg gctgtaaaaa ggtaatttta aagatggacc agtttggaaa tggacttgaa     720
   attgatcaag ctcggctagg aatgtgcaga cagcttgggg atgtattcac ggaagagaag     780
   tttcgttaca tgtgtattct ttcaggttgt gactacctgt catcactgcg tgggattgga     840
   ttagcaaagg catgcaaagt cctaagacta gccaataatc cagatatagt aaaggttatc     900
   aagaaaattg gacattatct caagatgaat atcacggtac cagaggatta catcaacggg     960
   tttattcggg ccaacaatac cttcctctat cagctagttt ttgatcccat caaaaggaaa    1020
   cttattcctc tgaacgccta tgaagatgat gttgatcctg aaacactaag ctacgctggg    1080
   caatatgttg atgattccat agctcttcaa atagcacttg gaaataaaga tataaatact    1140
   tttgaacaga tcgatgacta caatccagac actgctatgc ctgcccattc aagaagtcat    1200
   agttgggatg acaaaacatg tcaaaagtca gctaatgtta gcagcatttg gcataggaat    1260
   tactctccca gaccagagtc gggtactgtt tcagatgccc cacaattgaa ggaaaatcca    1320
   agtactgtgg gagtggaacg agtgattagt actaaagggt taaatctccc aaggaaatca    1380
   tccattgtga aaagaccaag aagtgcagag ctgtcagaag atgacctgtt gagtcagtat    1440
   tctctttcat ttacgaagaa gaccaagaaa aatagctctg aaggcaataa atcattgagc    1500
   ttttctgaag tgtttgtgcc tgacctggta aatggaccta ctaacaaaaa gagtgtaagc    1560
   actccaccta ggacgagaaa taaatttgca acatttttac aaaggaaaaa tgaagaaagt     1620
   ggtgcagttg tggttccagg gaccagaagc aggttttttt gcagttcaga ttctactgac     1680
   tgtgtatcaa acaaagtgag catccagcct ctggatgaaa ctgctgtcac agataaagag     1740
   aacaatctgc atgaatcaga gtatggagac caagaaggca agagactggt tgacacagat     1800
   gtagcacgta attcaagtga tgacattccg aataatcata ttccaggtga tcatattcca     1860
   gacaaggcaa cagtgtttac agatgaagag tcctactctt ttgagagcag caaatttaca     1920
   aggaccattt caccacccac tttgggaaca ctaagaagtt gttttagttg gtctggaggt     1980
   cttggagatt tttcaagaac gccgagcccc tctccaagca cagcattgca gcagttccga     2040
   agaaagagcg attcccccac ctctttgcct gagaataata tgtctgatgt gtcgcagtta     2100
   aagagcgagg agtccagtga cgatgagtct catcccttac gagaaggggc atgttcttca     2160
   cagtcccagg aaagtggaga attctcactg cagagttcaa atgcatcaaa gctttctcag     2220
   tgctctagta aggactctga ttcagaggaa tctgattgca atattaagtt acttgacagt     2280
   caaagtgacc agacctccaa gctatgttta tctcatttct caaaaaaaga cacacctcta     2340
   aggaacaagg ttcctgggct atataagtcc agttctgcag actctctttc tacaaccaag     2400
   atcaaacctc taggacctgc cagagccagt gggctgagca agaagccggc aagcatccag     2460
   aagagaaagc atcataatgc cgagaacaag ccggggttac agatcaaact caatgagctc     2520
   tggaaaaact ttggatttaa aaaagattct gaaaagcttc ctccttgtaa gaaacccctg     2580
   tccccagtca gagataacat ccaactaact ccagaagcgg aagaggatat atttaacaaa     2640
   cctgaatgtg gccgtgttca aagagcaa                                        2668